(19) Europäisches Patentamt
European Patent Office
Office européen des brevets

(11) EP 2 683 383 B1

# (12) EUROPEAN PATENT SPECIFICATION

(45) Date of publication and mention of the grant of the patent:
**29.11.2017 Bulletin 2017/48**

(21) Application number: **12710411.5**

(22) Date of filing: **09.03.2012**

(51) Int Cl.:
***A61K 31/495*** *(2006.01)* ***A61P 37/00*** *(2006.01)*

(86) International application number:
**PCT/US2012/028538**

(87) International publication number:
**WO 2012/125475 (20.09.2012 Gazette 2012/38)**

(54) **USE OF 3-(5-AMINO-2-METHYL-4-OXOQUINAZOLIN-3(4H)-YL)PIPERIDINE-2-6-DIONE IN TREATMENT OF IMMUNE-RELATED AND INFLAMMATORY DISEASES**

VERWENDUNG VON 3-(5-AMINO-2-METHYL-4-OXOCHINAZOLIN-3 (4H) -YL) PIPERIDIN-2-6-DION BEI DER BEHANDLUNG VON IMMUNBEDINGTEN UND ENTZÜNDLICHEN ERKRANKUNGEN

UTILISATION DE 3-(5-AMINO-2-MÉTHYL-4-OXOQUINAZOLIN-3(4H)-YL)PIPÉRIDINE-2-6-DIONE DANS LE TRAITEMENT DE MALADIES LIÉES AU SYSTÈME IMMUNITAIRE ET INFLAMMATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2011 US 201161451995 P**
**28.04.2011 US 201161480272 P**

(43) Date of publication of application:
**15.01.2014 Bulletin 2014/03**

(73) Proprietor: **Celgene Corporation**
**Summit, NJ 07901 (US)**

(72) Inventors:
- **GANDHI, Anita**
  **Bernardsville, NJ 07924 (US)**
- **SCHAFER, Peter, H.**
  **Somerset, NJ 08873 (US)**

(74) Representative: **Jones Day**
**Rechtsanwälte, Attorneys-at-Law, Patentanwälte**
**Prinzregentenstrasse 11**
**80538 München (DE)**

(56) References cited:
**WO-A2-2008/039489**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 683 383 B1

## Description

### 1. CROSS-REFERENCE TO RELATED APPLICATION

This application claims benefit of U.S. Provisional Patent Application No. 61/451,995, filed on March 11, 2011, and U.S. Provisional Patent Application No. 61/480,272, filed on April 28, 2011.

### 2. SEQUENCE LISTING

The present application is being filed with a Sequence Listing submitted as filename 12827-263-228_SeqListing.txt, of size 6,571 bytes, which was created on March 8, 2012. The Sequence Listing is incorporated herein by reference in its entirety.

### 3. FIELD

Provided herein are methods of treating, preventing, and/or managing diseases associated with lymphocytic activity, including activity of B cells and/or T cells, *e.g.*, immune-related diseases or inflammatory diseases, comprising administering Compound I or a pharmaceutically acceptable salt, solvate, hydrate, stereoisomer, tautomer, racemic mixture, co-crystal, clathrate, or polymorph thereof, where Compound I is 3-(5-amino-2-methyl-4-oxoquinazolin-3(4*H*)-yl)piperidine-2,6-dione. Pharmaceutical compositions and dosing regimens for such treatment, prevention, and/or management are also provided herein.

### 4. BACKGROUND

Inflammatory and immune-related diseases modulated by lymphocytic activity, including activity of B cells and/or T cells, such as lupus, scleroderma, Sjögren syndrome, ANCA-induced vasculitis, anti-phospholipid syndrome and myasthenia gravis, continue to be important medical problems.

Lupus or lupus erythematosus is a collection of autoimmune disorders that can cause chronic inflammation in various parts of the body, especially the skin, joints, blood, and kidneys. The body's immune system normally makes proteins called antibodies to protect the body against viruses, bacteria, and other foreign materials (i.e., antigens). In an autoimmune disorder such as lupus, the immune system loses its ability to tell the difference between antigens and its own cells and tissues and can make antibodies directed against its own cells and tissues to form immune complexes. These immune complexes can build up in the tissues and cause inflammation, injury to tissues and/or pain. The three most common types of lupus include systemic lupus erythematosus (SLE), cutaneous lupus erythematosus (CLE) and drug-induced lupus. More detailed descriptions of lupus or lupus erythematosus can be found in Wallace, 2000, The Lupus Book: A Guide for Patients and Their Families, Oxford University Press, Revised and Expanded Edition, which is incorporated by reference herein in its entirety.

Scleroderma is a rare disease with a stable incidence of approximately 19 cases per 1 million persons. The exact cause of scleroderma is unknown. Abnormalities involve autoimmunity and alteration of endothelial cell and fibroblast function. Systemic scleroderma usually begins with skin thickening, usually of the fingers, accompanied by Raynaud's phenomenon. Raynaud's disease typically precedes further manifestations of systemic scleroderma. Early in the disease the affected skin may be puffy and soft. The usual location of greatest skin thickening and hardening is the face, hands and fingers. Sclerodactyly is frequently present. Tendon friction rubs are often palpable on exam and can be painful. With more advanced disease, digital ulcers and auto-amputation may occur. Gastrointestinal dismotility is a feature, often manifested by heartburn, or by diarrhea with malabsorption or pseudo-obstruction. New onset hypertension or renal insufficiency are manifestations of the associated vascular injury. Heart failure or arrhythmia are also possible due to cardiac fibrosis. (Hachulla E, Launay D, Diagnosis and classification of systemic sclerosis, Clin Rev Allergy Immunol 2010; 40(2):78-83).

The major manifestations of scleroderma and in particular of systemic sclerosis are inappropriate excessive collagen synthesis and deposition, endothelial dysfunction, spasm, collapse and obliteration by fibrosis. In terms of diagnosis, an important clinical parameter is skin thickening proximal to the metacarpophalangeal joints. Raynaud's phenomenon is a frequent, almost universal component of scleroderma. It is diagnosed by color changes of the skin upon cold exposure. Ischemia and skin thickening are symptoms of Raynaud's disease.

There remains a need for prophylactic or therapeutic drugs that can be used to treat or prevent immune-related and inflammatory diseases, including lupus, scleroderma, Sjögren syndrome, ANCA-induced vasculitis, anti-phospholipid syndrome and myasthenia gravis.

## 5. SUMMARY

Provided herein are methods of treating, managing, ameliorating and/or preventing diseases, disorders and/or conditions associated with immune-related and inflammatory diseases comprising administering a therapeutically effective amount of a compound of formula I

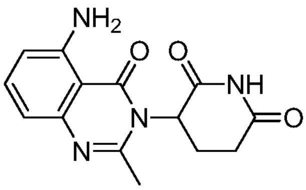

Compound I,

or a pharmaceutically acceptable salt, solvate, hydrate, stereoisomer, tautomer, racemic mixture, co-crystal, clathrate, or polymorph thereof.

In one embodiment, the compound is 3-(5-amino-2-methyl-4-oxoquinazolin-3(4*H*)-yl)piperidine-2,6-dione.

In certain embodiments, the disease is selected from lupus, scleroderma, Sjögren syndrome, ANCA-induced vasculitis, anti-phospholipid syndrome and myasthenia gravis.

In one embodiment, provided herein are methods of modulating, *e.g.*, reducing, lymphocytic activity, including activity of B cells and/or T cells, comprising contacting B cell and/or T cell with an effective amount of Compound I.

Also provided herein are pharmaceutical compositions, single unit dosage forms, and kits suitable for use in treating, preventing, ameliorating and/or managing diseases, disorders and/or conditions associated immune-related and inflammatory diseases, which comprise Compound I, optionally in combination with one or more other therapeutic agents.

In certain embodiments, Compound I is administered in combination with one or more therapeutic agents, *i.e.,* pharmaceutical agents that are modulators of lymphocytic activity, including activity of B cells and/or T cells activity. The combinations encompass simultaneous as well as sequential administration.

## 6. BRIEF DESCRIPTION OF DRAWINGS

FIG. 1 illustrates the effect of 3-(5-amino-2-methyl-4-oxoquinazolin-3(4*H*)-yl)piperidine-2,6-dione on cytokine and chemokine production in anti-CD3- stimulated human T cells, expressed as absolute amount produced.

FIG. 2 illustrates the effect of 3-(5-amino-2-methyl-4-oxoquinazolin-3(4*H*)-yl)piperidine-2,6-dione on cytokine and chemokine production in anti-CD3- stimulated human T cells, expressed as percentage of control.

FIG. 3 illustrates inhibition of production of cytokine and chemokine production in lipopolysaccharide-stimulated peripheral blood mononuclear cells by 3-(5-amino-2-methyl-4-oxoquinazolin-3(4*H*)-yl)piperidine-2,6-dione.

FIG. 4 illustrates enhancement of production of cytokine and chemokine production in lipopolysaccharide-stimulated peripheral blood mononuclear cells by 3-(5-amino-2-methyl-4-oxoquinazolin-3(4*H*)-yl)piperidine-2,6-dione.

FIG. 5 illustrates enhancement of NK cell IFN-gamma production in response to immobilized IgG and IL-2, expressed as absolute amount produced, for 3-(5-amino-2-methyl-4-oxoquinazolin-3(4*H*)-yl)piperidine-2,6-dione.

FIG. 6 illustrates enhancement of NK cell IFN-gamma production in response to immobilized IgG and IL-2, expressed as percentage of amount of IFN-gamma produced in the presence of 1 $\mu$m pomalidomide, for 3-(5-amino-2-methyl-4-oxoquinazolin-3(4*H*)-yl)piperidine-2,6-dione.

FIG. 7 illustrates the effect of 3-(5-amino-2-methyl-4-oxoquinazolin-3(4*H*)-yl)piperidine-2,6-dione on NK-cell mediated ADCC against Rituximab coated lymphoma cells.

FIG. 8 illustrates hematoxylin and eosin stained skin section photomicrographs showing dermal thickness of lesional skin in the bleomycin dermal fibrosis mouse model (prevention of inflammation driven fibrosis).

FIG. 9 illustrates hematoxylin and eosin stained skin section photomicrographs showing dermal thickness of lesional skin in the bleomycin dermal fibrosis mouse model (regression of established fibrosis).

## 7. DETAILED DESCRIPTION

Unless defined otherwise, all technical and scientific terms used herein have the same meaning as is commonly understood by one of ordinary skill in the art. All patents, applications, published applications and other publications are incorporated by reference in their entirety. In the event that there is a plurality of definitions for a term herein, those in

this section prevail unless stated otherwise.

As used herein, and unless otherwise indicated, the terms "treat," "treating" and "treatment" refer to alleviating or reducing the severity of a disease or a symptom associated with the disease or condition being treated.

As used herein, "prevent", "prevention" and other forms of the word include the inhibition of onset or progression of a disease or disorder or a symptom of the particular disease or disorder. In some embodiments, subjects with familial history of cancer are candidates for preventive regimens. Generally, in the context of cancer, the term "preventing" refers to administration of the drug prior to the onset of signs or symptoms of a cancer, particularly in subjects at risk of cancer.

As used herein, and unless otherwise indicated, the term "managing" encompasses preventing the recurrence of the particular disease or disorder in a subject who had suffered from it, lengthening the time a subject who had suffered from the disease or disorder remains in remission, reducing mortality rates of the subjects, and/or maintaining a reduction in severity or avoidance of a symptom associated with the disease or condition being managed.

As used herein, "subject" means an animal, typically a mammal, including a human being. As used herein, "patient" means a human subject.

As used herein, and unless otherwise specified, the terms "therapeutically effective amount" and "effective amount" of a compound refer to an amount sufficient to provide a therapeutic benefit in the treatment, prevention and/or management of a disease, to delay or minimize one or more symptoms associated with the disease or disorder to be treated. The terms "therapeutically effective amount" and "effective amount" can encompass an amount that improves overall therapy, reduces or avoids symptoms or causes of disease or disorder or enhances the therapeutic efficacy of another therapeutic agent.

As used herein, and unless otherwise specified, the term "prophylactically effective amount" of a compound is an amount sufficient to prevent a disease or condition, or one or more symptoms associated with the disease or condition, or prevent its recurrence. A prophylactically effective amount of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

As used herein and unless otherwise indicated, the term "pharmaceutically acceptable salt" includes, but is not limited to, a salt of an acidic group that can be present in the compounds provided herein. Under certain acidic conditions, the compound can form a wide variety of salts with various inorganic and organic acids. The acids that can be used to prepare pharmaceutically acceptable salts of such basic compounds are those that form salts comprising pharmacologically acceptable anions including, but not limited to, acetate, benzenesulfonate, benzoate, bicarbonate, bitartrate, bromide, calcium edetate, camsylate, carbonate, chloride, bromide, iodide, citrate, dihydrochloride, edetate, edisylate, estolate, esylate, fumarate, gluceptate, gluconate, glutamate, glycollylarsanilate, hexylresorcinate, hydrabamine, hydroxynaphthoate, isethionate, lactate, lactobionate, malate, maleate, mandelate, methanesulfonate (mesylate), methylsulfate, muscate, napsylate, nitrate, pantothenate, phosphate/diphosphate, polygalacturonate, salicylate, stearate, succinate, sulfate, tannate, tartrate, teoclate, triethiodide, and pamoate.

As used herein and unless otherwise indicated, the term "hydrate" means a compound provided herein or a salt thereof, further including a stoichiometric or non-stoichiometric amount of water bound by non-covalent intermolecular forces. The hydrates can be crystalline or non-crystalline.

As used herein and unless otherwise indicated, the term "solvate" means a solvate formed from the association of one or more solvent molecules to compound provided herein. The term "solvate" includes hydrates (e.g., monohydrate, dihydrate, trihydrate, tetrahydrate, and the like). The solvates can be crystalline or non-crystalline.

As used herein, and unless otherwise specified, the term "stereoisomer" encompasses all enantiomerically/stereomerically pure and enantiomerically/stereomerically enriched compounds provided herein.

As used herein, and unless otherwise indicated, the term "stereomerically pure" or "enantiomerically pure" means that a compound comprises one stereoisomer and is substantially free of its counter stereoisomer or enantiomer. For example, a compound is stereomerically or enantiomerically pure when the compound contains 80%, 90%, or 95% or more of one stereoisomer and 20%, 10%, or 5% or less of the counter stereoisomer. In certain cases, a compound provided herein is considered optically active or stereomerically/enantiomerically pure (*i.e.,* substantially the *R*-form or substantially the *S*-form) with respect to a chiral center when the compound is about 80% ee (enantiomeric excess) or greater, preferably, equal to or greater than 90% ee with respect to a particular chiral center, and more preferably 95% ee with respect to a particular chiral center.

As used herein, and unless otherwise indicated, the term "stereomerically enriched" or "enantiomerically enriched" encompasses racemic mixtures as well as other mixtures of stereoisomers of compounds provided herein (*e.g.*, R/S = 30/70, 35/65, 40/60, 45/55, 55/45, 60/40, 65/35 and 70/30).

The terms "co-administration" and "in combination with" include the administration of two or more therapeutic agents (for example, Compound I or a composition provided herein and another modulator of lymphocytic activity, including activity of B cells and/or T cells activity or other active agent) either simultaneously, concurrently or sequentially with no specific time limits. In one embodiment, Compound I and at least one other agent are present in the cell or in

the subject's body at the same time or exert their biological or therapeutic effect at the same time. In one embodiment, the therapeutic agent(s) are in the same composition or unit dosage form. In another embodiment, the therapeutic agent(s) are in separate compositions or unit dosage forms.

A "B cell" is a lymphocyte that matures within the bone marrow, and includes a naive B cell, memory B cell, or effector B cell (plasma cells). The B cell herein may be a normal or non-malignant B cell.

A "T cell" is a lymphocyte that matures in thymus, and includes a helper T cell, a memory T cell, and a cytotoxic T cell.

As used herein "overall survival" refers to the time from randomization until death from any cause, and is measured in the intent-to-treat population. Overall survival can be evaluated in randomized controlled studies.

As used herein "objective response rate" refers to the proportion of patients with reduced predefined scleroderma symptoms at the end of a predefined period of time. Response duration is usually measured from the time of initial response until documented scleroderma progression.

As used herein "time to progression" means the time from randomization until objective scleroderma progression. In certain embodiments, time to progression does not include deaths.

As used herein "progression-free survival" means the time from randomization until objective scleroderma progression or death.

As used herein "time-to-treatment failure" means any endpoint(s) measuring time from randomization to discontinuation of treatment for any reason, including disease progression, treatment toxicity, and death.

As used herein "mortality" means a measure of the number of deaths in a given population.

As used herein "respiratory mortality" means patients who die from acute hypoxemia or other specific respiratory deterioration resulting in death such as need for mechanical ventilation leading to death, respiratory arrest, or any other event in a subject deemed to be respiratory in nature.

As used herein "respiratory hospitalization" means those hospitalized for deterioration in pulmonary status as documented by patient hospital admission notes or other medical opinion.

As used herein "modified Rodnan skin score" means a validated numerical scoring system to assess dermal skin thickness.

As used herein "skin thickness" means hard or indurated skin that can be evaluated using a variety of techniques including durometer and mRSS

As used herein "skin induration" means skin that is hardened, red, inflamed, thickened or tender.

As used herein "dermatology quality of life index" means an evaluation of quality or life related to the skin symptoms for a patient having scleroderma.

As used herein "pulmonary function" means any measurement of forced expiratory flow, forced vital capacity, FEV 25-75%, lung volumes or vital capacity.

As used herein "carbon monoxide diffusing capacity" means an assessment of the uptake of carbon monoxide across the alveolar-capillary membrane. It can be a proxy for the measurement of the lungs ability to transfer oxygen from the lungs to the blood stream.

As used herein "Mahler Dyspnea index" means an instrument that provides clinical measurement of shortness of breath.

As used herein "Saint George's Respiratory Questionnaire score" means an instrument that measures quality of life in patients with pulmonary disease.

As used herein "UCLA scleroderma clinical trial consortium gastrointestinal tract score" means a questionnaire administered to patients having scleroderma to evaluate gastrointestinal symptoms associated with scleroderma (systemic sclerosis).

As used herein "flow-mediated dilatation" means any measurement of vascular endothelial function in a patient having scleroderma.

As used herein "six minute walk distance" means any evaluation of the distance a patient having scleroderma can walk within 6 minutes or any standardized procedure to evaluate ability to walk for a fixed period of time or distance.

As used herein, pomalidomide refers to the following compound:

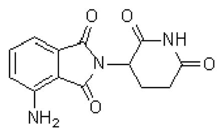

.

## 7.1 COMPOUND I

In certain embodiments, Compound I for use in the methods provided herein, including the combination therapy,

and in compositions provided herein is a compound of formula:

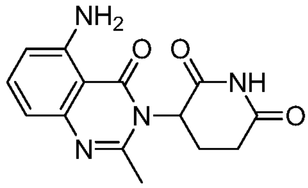

Compound I,

or a pharmaceutically acceptable salt, solvate, hydrate, stereoisomer, tautomer, racemic mixture, co-crystal, clathrate, or polymorph thereof.

In one embodiment, the compound is 3-(5-amino-2-methyl-4-oxoquinazolin-3(4*H*)-yl)piperidine-2,6-dione.

Compound I or a pharmaceutically acceptable salt, solvate, hydrate, stereoisomer, tautomer, racemic mixture, co-crystal, clathrate, or polymorph thereof can be prepared by methods known to one of skill in the art, for example, according to the procedure described in US Patent 7,635,700 and US Provisional App. No. 61/451,806.

In certain embodiments, the compound of Formula I is a solid. In certain embodiments, the compound of Formula I is hydrated. In certain embodiments, the compound of Formula I is solvated. In certain embodiments, the compound of Formula I is anhydrous. In certain embodiments, the compound of Formula I is nonhygroscopic.

In certain embodiments, the solid compound of Formula I is amorphous. In certain embodiments, the solid compound of Formula I is crystalline. In certain embodiments, the solid compound of Formula I is in a crystalline form described in U.S. Provisional Pat. App. No. 61/451,806, filed March 11, 2011, which is incorporated herein by reference in its entirety.

The solid forms of the compound of Formula I can be prepared according to the methods described in the disclosure of U.S. Provisional Pat. App. No. 61/451,806. The solid forms can be also prepared according to other methods apparent to those of skill in the art.

In certain embodiments, the compound of Formula I is a hydrochloride salt of 3-(5-amino-2-methyl-4-oxo-4*H*-quinazolin-3-yl)-piperidine-2,6-dione, or an enantiomer or a mixture of enantiomers thereof; or a pharmaceutically acceptable solvate, hydrate, co-crystal, clathrate, or polymorph thereof. In certain embodiments, the hydrochloride salt is a solid. In certain embodiments, the hydrochloride salt is anhydrous. In certain embodiments, the hydrochloride salt is nonhygroscopic. In certain embodiments, the hydrochloride salt is amorphous. In certain embodiments, the hydrochloride salt is crystalline. In certain embodiments, the hydrochloride salt is in crystalline Form A.

The hydrochloride salt of the compound of Formula I and solid forms thereof can be prepared according to the methods described in the disclosure of U.S. Provisional Pat. App. No. 61/451,806. The hydrochloride salt the solid forms thereof can be also prepared according to other methods apparent to those of skill in the art.

The compound of Formula I provided herein contains one chiral center, and can exist as a mixture of enantiomers, *e.g.*, a racemic mixture. This disclosure encompasses the use of stereomerically pure forms of such a compound, as well as the use of mixtures of those forms. For example, mixtures comprising equal or unequal amounts of the enantiomers of the compound of Formula I provided herein may be used in methods and compositions disclosed herein. These isomers may be asymmetrically synthesized or resolved using standard techniques such as chiral columns or chiral resolving agents. *See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of the structure.

## 7.2 METHODS OF TREATMENT

Provided herein are methods of treating, preventing, and/or managing diseases, disorders and/or conditions associated immune-related and inflammatory diseases comprising administering a therapeutically effective amount of Compound I or a pharmaceutically acceptable salt, solvate, hydrate, stereoisomer, tautomer or racemic mixtures thereof to a patient in need thereof. In certain embodiments, the disease is selected from lupus, scleroderma, Sjögren syndrome, ANCA-induced vasculitis, anti-phospholipid syndrome and myasthenia gravis. In certain embodiments, the disease is lupus or scleroderma.

The sensitivity of Compound I or a pharmaceutically acceptable salt, solvate, hydrate, stereoisomer, tautomer

or racemic mixtures thereof can be studied in various *in vivo* and *in vitro* assays, including animal models known to one of skill in the art for immune-related and inflammatory diseases, including, but not limited to MRL/MpJ-Faslpr/J mouse model of systemic lupus erythematosus, NZBWF1/J mouse model of systemic lupus erythematosus, bleomycin-induced skin fibrosis model, and murine tight skin-1 (Tsk-1) mouse model.

**7.2.1** Treatment of Scleroderma

In certain embodiments, provided herein are methods of treating, preventing, and/or managing scleroderma or a symptom thereof, comprising administering a therapeutically effective amount of Compound I or a pharmaceutically acceptable salt, solvate, hydrate, stereoisomer, tautomer or racemic mixtures thereof to a patient having scleroderma.

In certain embodiments, provided herein are methods of preventing scleroderma or a symptom thereof, comprising administering an effective amount of Compound I or a pharmaceutically acceptable salt, solvate, hydrate, stereoisomer, tautomer or racemic mixtures thereof to a patient at risk of having scleroderma.

In certain embodiments, the scleroderma is localized, systemic, limited or diffuse scleroderma.

In certain embodiments, the systemic scleroderma comprises CREST syndrome (Calcinosis, Raynaud's syndrome, esophagaeal dysfunction or dysmotility, sclerodactyly, telangiectasia). Scleroderma is also known as systemic sclerosis or progressive systemic sclerosis. In certain embodiments, provided herein are methods of treating or preventing Raynaud's disease or syndrome. In certain embodiments, systemic sclerosis comprises scleroderma lung disease, scleroderma renal crisis, cardiac manifestations, muscular weakness (including fatigue or limited CREST), gastrointestinal dysmotility and spasm, and abnormalities in the central, peripheral and autonomic nervous system (including carpal tunnel syndrome followed by trigeminal neuralgia). It also includes general disability, including depression, and impact on quality of life.

In certain embodiments, limited scleroderma is limited to the hands, the face, neck, or combinations thereof.

In certain embodiments, diffuse scleroderma comprises skin tightening and also occurs above the wrists (or elbows). In certain embodiments, the diffuse systemic sclerosis is sine scleroderma, comprising internal organ fibrosis, but no skin tightening; or familial progressive systemic sclerosis.

In one embodiment, scleroderma is not associated with wasting, such as disease-related wasting.

In one embodiment, provided herein are methods for the reduction, inhibition, or prevention of one or more of the following symptoms of scleroderma: (i) gradual hardening, thickening, and tightening of the skin (e.g., in extremities, such as hands, face, and feet); (ii) skin discoloration; (iii) numbness of extremities; (iv) shiny skin; (v) small white lumps under the surface of the skin that erupt into a chalky white fluid; (vi) Raynaud's esophagaeal dysfunction (pain, numbness, and/or color changes in the hands caused by spasm of the blood vessels upon exposure to cold or emotional stress); (vii) telangiectasia (red spots on, e.g., the hands, palms, forearms, face, and lips); (viii) pain and/or stiffness of the joints; (ix) swelling of the hands and feet; (x) itching of the skin; (xi) stiffening and curling of the fingers; (xii) ulcers (sores) on the outside of certain joints, such as knuckles and elbows; (xiii) digestive problems, such as heartburn, difficulty in swallowing, diarrhea, irritable bowel, and constipation; (xiv) fatigue and weakness; (xv) shortness of breath; (xvi) arthritis; (xvii) hair loss; (xviii) internal organ problems; (xix) digital ulcers; or (xx) digital auto-amputation, comprising administering an effective amount of Compound I to a patient in need thereof.

Without being bound to any particular theory, it is believed that Compound I or a pharmaceutically acceptable salt, solvate, hydrate, stereoisomer, tautomer or racemic mixtures thereof enhances Th1 immune response, and suppresses Th2 immune response, which may result in anti-fibrotic effects in the skin.

Further provided herein are methods for improving or reducing the skin thickness of a patient having scleroderma comprising administering an effective amount of Compound I or a pharmaceutically acceptable salt, solvate, hydrate, stereoisomer, tautomer or racemic mixtures thereof to the patient. In one embodiment, the skin thickness is reduced by about 20%, about 25%, about 30%, about 40%, about 50%, about 60%, about 70% about 80%, about 90% or more.

Further provided herein are methods for achieving one or more clinical endpoints associated with scleroderma comprising administering an effective amount of Compound I or a pharmaceutically acceptable salt, solvate, hydrate, stereoisomer, tautomer or racemic mixtures thereof to a patient in need thereof.

Further provided herein are methods for increasing the overall survival, objective response rate, time to progression, progression-free survival and/or time-to-treatment failure of a patient having scleroderma comprising administering an effective amount of Compound I or a pharmaceutically acceptable salt, solvate, hydrate, stereoisomer, tautomer or racemic mixtures thereof to the patient.

Further provided herein are methods for decreasing mortality, respiratory mortality and/or respiratory hospitalization of a patient having scleroderma comprising administering an effective amount of Compound I or a pharmaceutically acceptable salt, solvate, hydrate, stereoisomer, tautomer or racemic mixtures thereof to the patient.

Further provided herein are methods for improving the modified Rodnan skin score of a patient having scleroderma comprising administering an effective amount of Compound I or a pharmaceutically acceptable salt, solvate, hydrate, stereoisomer, tautomer or racemic mixtures thereof to the patient. In one embodiment, the improvement in

modified Rodnan skin score is 5, 10, 15 or 20 points or more.

Further provided herein are methods for improving or reducing the skin thickness of a patient having scleroderma comprising administering an effective amount of Compound I or a pharmaceutically acceptable salt, solvate, hydrate, stereoisomer, tautomer or racemic mixtures thereof to the patient. In one embodiment, the skin thickness is reduced by about 20%, about 25%, about 30%, about 40%, about 50%, about 60%, about 70% about 80%, about 90% or more.

Further provided herein are methods for improving or reducing skin induration of a patient having scleroderma comprising administering an effective amount of Compound I or a pharmaceutically acceptable salt, solvate, hydrate, stereoisomer, tautomer or racemic mixtures thereof to the patient.

Further provided herein are methods for improving the dermatology quality of life index of a patient having scleroderma comprising administering an effective amount of Compound I or a pharmaceutically acceptable salt, solvate, hydrate, stereoisomer, tautomer or racemic mixtures thereof to the patient.

Further provided herein are methods for improving the pulmonary function of a patient having scleroderma comprising administering an effective amount of Compound I or a pharmaceutically acceptable salt, solvate, hydrate, stereoisomer, tautomer or racemic mixtures thereof to the patient.

Further provided herein are methods for improving the carbon monoxide diffusing capacity of a patient having scleroderma comprising administering an effective amount of Compound I or a pharmaceutically acceptable salt, solvate, hydrate, stereoisomer, tautomer or racemic mixtures thereof to the patient. In one embodiment, the carbon monoxide diffusing capacity of a patient is improved by an improvement in the diffusing capacity of the lung for carbon monoxide ($D_L$co) of about 10%, about 20%, about 25%, about 30%, about 40%, about 50%, about 60%, about 70% about 80%, about 90% or more.

Further provided herein are methods for improving the Mahler Dyspnea index of a patient having scleroderma comprising administering an effective amount of Compound I or a pharmaceutically acceptable salt, solvate, hydrate, stereoisomer, tautomer or racemic mixtures thereof to the patient. In one embodiment, the improvement in Mahler Dyspnea index is 4, 5, 6, 7, 8, 9 or 10 points or more.

Further provided herein are methods for improving the Saint George's Respiratory Questionnaire score of a patient having scleroderma comprising administering an effective amount of Compound I or a pharmaceutically acceptable salt, solvate, hydrate, stereoisomer, tautomer or racemic mixtures thereof to the patient..In one embodiment, the improvement in Saint George's Respiratory Questionnaire score is 4, 8, 12, 16, 20, 24, 28, 32, 36, 40, 44, 48, 52 points or more.

Further provided herein are methods for improving the UCLA scleroderma clinical trial consortium gastrointestinal tract score of a patient having scleroderma comprising administering an effective amount of Compound I or a pharmaceutically acceptable salt, solvate, hydrate, stereoisomer, tautomer or racemic mixtures thereof to the patient.

Further provided herein are methods for treating or preventing digital ulcer of a patient or patient population having scleroderma comprising administering an effective amount of Compound I or a pharmaceutically acceptable salt, solvate, hydrate, stereoisomer, tautomer or racemic mixtures thereof to the patient.

Further provided herein are methods improving flow-mediated dilatation of a patient having scleroderma comprising administering an effective amount of Compound I or a pharmaceutically acceptable salt, solvate, hydrate, stereoisomer, tautomer or racemic mixtures thereof to the patient.

Further provided herein are methods improving or increasing the six minute walk distance of a patient having scleroderma comprising administering an effective amount of Compound I or a pharmaceutically acceptable salt, solvate, hydrate, stereoisomer, tautomer or racemic mixtures thereof to the patient. In one embodiment, the improvement in the six minute walk distance is about 200 meters, about 250 meters, about 300 meters, about 350 meters, about 400 meters or more.

### **7.2.2** Treatment of Lupus Erythematosus

In certain embodiments, provided herein are methods of treating, preventing, and/or managing lupus erythematosus or a symptom thereof, comprising administering a therapeutically effective amount of Compound I or a pharmaceutically acceptable salt, solvate, hydrate, stereoisomer, tautomer or racemic mixtures thereof to a patient having lupus erythematosus.

In one embodiment, provided herein are methods of preventing lupus erythematosus or a symptom thereof, comprising administering an effective amount of Compound I or a pharmaceutically acceptable salt, solvate, hydrate, stereoisomer, tautomer or racemic mixtures thereof to a patient at risk of having lupus erythematosus.

In certain embodiments, provided herein are methods for treating, preventing, and/or managing systemic lupus erythematosus (SLE), cutaneous lupus erythematosus (CLE) or drug-induced lupus.

The phrase "Systemic lupus erythematosus" is interchangeably used herein with SLE and lupus and refers to all manifestations of the disease as known in the art (including remissions and flares). In SLE, abnormal hyperactivity of B lymphocytes and massive abnormal production of immunoglobulin gamma (IgG) auto-antibodies play a key role.

This pathological process results in sequestration and destruction of Ig-coated cells, fixation and cleaving of complement proteins, and release of chemotaxins, vasoactive peptides and destructive enzymes into tissues (Hahn BH. Systemic Lupus Erythematosus. In: Kasper DL, Braunwald E, Fauci AS, Hauser SL, Longo DL, Jameson, JL, editors. In: Harrison's Principles of Internal Medicine (16th edition). New York (US): McGraw-Hill; 2005. pp.1960-1967).

Symptoms of SLE vary from person to person, and may come and go. In most patients, the symptoms include joint pain and swelling. Frequently affected joints are the fingers, hands, wrists, and knees. Some patients develop arthritis. Other common symptoms include: chest pain when taking a deep breath, fatigue, fever with no other cause, general discomfort, uneasiness, or ill feeling (malaise), hair loss, mouth sores, swollen lymph nodes, sensitivity to sunlight, skin rash -a "butterfly" rash over the cheeks and bridge of the nose affects about half of people with SLE, in some patients, the rash gets worse in sunlight, and the rash may also be widespread.

Other symptoms depend on what part of the body is affected, and may include the following:

Brain and nervous system: headaches, numbness, tingling, seizures, vision problems, personality changes,
Digestive tract: abdominal pain, nausea, and vomiting,
Heart: abnormal heart rhythms (arrhythmias),
Lung: coughing up blood and difficulty breathing, and
Skin: patchy skin color, fingers that change color when cold (Raynaud's phenomenon).

Some patients only have skin symptoms. This is called discoid lupus.

In one embodiment, provided herein are methods of treating moderate, severe, or very severe SLE. The term "severe SLE" as used herein refers to an SLE condition where the patient has one or more severe or life-threatening symptoms (such as hemolytic anemia, extensive heart or lung involvement, kidney disease, or central nervous system involvement).

Further provided herein are methods for achieving one or more clinical endpoints associated with SLE comprising administering an effective amount of Compound I or a pharmaceutically acceptable salt, solvate, hydrate, stereoisomer, tautomer or racemic mixtures thereof to a patient in need thereof.

Further provided herein are methods for increasing the overall survival, objective response rate, time to progression, progression-free survival and/or time-to-treatment failure of a patient having SLE comprising administering an effective amount of Compound I or a pharmaceutically acceptable salt, solvate, hydrate, stereoisomer, tautomer or racemic mixtures thereof to the patient.

In certain embodiment, Compound I or a pharmaceutically acceptable salt, solvate, hydrate, stereoisomer, tautomer or racemic mixtures thereof acts as an inhibitor of primary human memory CD19+ B-cell differentiation to the plasmablast stage. Without being bound to any particular theory, it is believed that Compound I or a pharmaceutically acceptable salt, solvate, hydrate, stereoisomer, tautomer or racemic mixtures thereof blocks cells at a premature stage thereby decreasing the numbers of plasmablasts that are capable of producing high levels of immunoglobulin. A functional consequence of this effect is reduced immunoglobulin G (IgG) and immunoglobulin M (IgM) production in these differentiation cultures.

In certain embodiments, Compound I or a pharmaceutically acceptable salt, solvate, hydrate, stereoisomer, tautomer or racemic mixtures thereof inhibits of the ability of primary human memory CD19+ B-cells to differentiate to the plasmablast stage. In certain embodiments, Compound I or a pharmaceutically acceptable salt, solvate, hydrate, stereoisomer, tautomer or racemic mixtures thereof has no significant effect on mature CD138+ plasma cells in short term cultures. In certain embodiments, Compound I or a pharmaceutically acceptable salt, solvate, hydrate, stereoisomer, tautomer or racemic mixtures thereof inhibits B cell differentiation factors including interferon regulatory factor 4 (IRF4), lymphocyte-induced maturation protein (BLIMP), X-box-protein-1 (XBP-1) and B cell lymphoma 6 (Bcl6).

### **7.2.3** Treatment of Other Immune-Related Diseases or Disorders

Further provided herein are methods of treating, managing, or preventing other immune-related diseases or conditions using Compound I or a pharmaceutically acceptable salt, solvate, hydrate, stereoisomer, tautomer or racemic mixtures thereof. In certain embodiments, for example, provided herein is a method of treating an individual having a disease or disorder, wherein the disease or disorder is caused by, or is associated with, an inappropriate or undesirable immune response, *e.g.*, a disease, disorder or condition that can be treated beneficially by immunosuppression, comprising administering to the individual Compound I or a pharmaceutically acceptable salt, solvate, hydrate, stereoisomer, tautomer or racemic mixtures thereof.

In various specific embodiments, said immune-related disease is one or more of selected from Sjögren syndrome, ANCA-induced vasculitis, anti-phospholipid syndrome, myasthenia gravis, Addison's disease, alopecia areata, ankylosing spondylitis, antiphospholipid antibody syndrome, antiphospholipid syndrome (primary or secondary), asthma, autoimmune gastritis, autoimmune hemolytic anemia, autoimmune hepatitis, autoimmune inner ear disease, autoimmune

lymphoproliferative disease, autoimmune thrombocytopenic purpura, Balo disease, Behcet's disease, bullous pemphigoid, cardiomyopathy, celiac disease, Chagas disease, chronic inflammatory demyelinating polyneuropathy, cicatrical pemphigoid (e.g., mucous membrane pemphigoid), cold agglutinin disease, degos disease, dermatitis hepatiformis, essential mixed cryoglobulinemia, Goodpasture's syndrome, Graves' disease, Guillain-Barre syndrome, Hashimoto's thyroiditis (Hashimoto's disease; autoimmune thyroditis), idiopathic pulmonary fibrosis, idiopathic thrombocytopenia purpura, IgA nephropathy, juvenile arthritis, lichen planus, Ménière disease, mixed connective tissue disease, morephea, narcolepsy, neuromyotonia, pediatric autoimmune neuropsychiatric disorders (PANDAs), pemphigus vulgaris, pernicious anemia, polyarteritis nodosa, polychondritis, polymyalgia rheumatica, primary agammaglobulinemia, primary biliary cirrhosis, Raynaud disease (Raynaud phenomenon), Reiter's syndrome, relapsing polychondritis, rheumatic fever, Sjogren's syndrome, stiff-person syndrome (Moersch-Woltmann syndrome), Takayasu's arteritis, temporal arteritis (giant cell arteritis), uveitis, vasculitis (e.g., vasculitis not associated with lupus erythematosus), vitiligo, and/or Wegener's granulomatosis.

## 7.3 DOSAGES AND DOSING AMOUNTS

The dose of Compound I or a pharmaceutically acceptable salt, solvate, hydrate, stereoisomer, tautomer or racemic mixtures thereof to be administered to a patient is rather widely variable and can be subject to the judgment of a health-care practitioner. Doses of Compound I or a pharmaceutically acceptable salt, solvate, hydrate, stereoisomer, tautomer or racemic mixtures thereof vary depending on factors such as: specific indication to be treated, prevented, or managed; age and condition of a patient; and amount of second active agent used, if any. In general, Compound I or a pharmaceutically acceptable salt, solvate, hydrate, stereoisomer, tautomer or racemic mixtures thereof can be administered one to four or more times a day in a dose of about 0.005 mg/kg of a patient's body weight to about 10 mg/kg of a patient's body weight in a patient, but the above dosage may be properly varied depending on the age, body weight and medical condition of the patient and the type of administration. In one embodiment, the dose is about 0.01 mg/kg of a patient's body weight to about 5 mg/kg of a patient's body weight, about 0.05 mg/kg of a patient's body weight to about 1 mg/kg of a patient's body weight, about 0.1 mg/kg of a patient's body weight to about 0.75 mg/kg of a patient's body weight or about 0.25 mg/kg of a patient's body weight to about 0.5 mg/kg of a patient's body weight.

In one embodiment, one dose is given per day. In any given case, the amount of Compound I or a pharmaceutically acceptable salt, solvate, hydrate, stereoisomer, tautomer or racemic mixtures thereof administered will depend on such factors as the solubility of the active component, the formulation used and the route of administration. In one embodiment, application of a topical concentration provides intracellular exposures or concentrations of about 0.01 - 10 μM.

In certain embodiments, Compound I or a pharmaceutically acceptable salt, solvate, hydrate, stereoisomer, tautomer or racemic mixtures thereof is used in an amount of from about 0.1 mg to about 1000 mg per day, and can be adjusted in a conventional fashion (*e.g.,* the same amount administered each day of the treatment, prevention or management period), in cycles (*e.g.,* one week on, one week off), or in an amount that increases or decreases over the course of treatment, prevention, or management. In other embodiments, the dose can be from about 1 mg to about 300 mg, from about 0.1 mg to about 150 mg, from about 1 mg to about 200 mg, from about 10 mg to about 100 mg, from about 0.1 mg to about 50 mg, from about 1 mg to about 50 mg, from about 10 mg to about 50 mg, from about 20 mg to about 30 mg, or from about 1 mg to about 20 mg.

## 7.4 COMBINATION THERAPY

Compound I or a pharmaceutically acceptable salt, solvate, hydrate, stereoisomer, tautomer or racemic mixtures thereof can be combined with other pharmacologically active compounds ("second active agents") in methods and compositions provided herein. Certain combinations may work synergistically in the treatment of particular types diseases or disorders, and conditions and symptoms associated with such diseases or disorders. Compound I or a pharmaceutically acceptable salt, solvate, hydrate, stereoisomer, tautomer or racemic mixtures thereof can also work to alleviate adverse effects associated with certain second active agents, and vice versa.

One or more second active ingredients or agents can be used in the methods and compositions provided herein. Second active agents can be large molecules (e.g., proteins) or small molecules (e.g., synthetic inorganic, organometallic, or organic molecules).

In another embodiment, the method of treatment provided herein comprises the administration of a second therapeutic agent, wherein the second therapeutic agent is an anti-inflammatory drug, *e.g.,* a steroidal anti-inflammatory drug, or a non-steroidal anti-inflammatory drug (NSAID), acetaminophen, naproxen, ibuprofen, acetylsalicylic acid, and the like. In a more specific embodiment in which an NSAID is administered, a proton pump inhibitor (PPI), *e.g.,* omeprazole may also administered. In one embodiment, the antiinflammatory agent is a corticosteroid. In another embodiment, the antiinflammatory agent is colchicine.

In another embodiment, the second therapeutic agent is an immunomodulatory compound or an immunosuppressant compound such as azathioprine (Imuran™, Azasan™), methotrexate (Rheumatrex™, Trexall™), penicillamine (Depen™, Cuprimine™), cyclophosphamide (Cytoxan™), mycophenalate (CellCept™, Myfortic™), bosentan (Tracleer®), prednisone (Deltasone™, Liquid Pred™), and a PDE5 inhibitor. In another embodiment, where the affected individual has digital ulcerations and pulmonary hypertension, a vasodilator such as prostacyclin (iloprost) may be administered.

In another embodiment, the second therapeutic agent is an inhibitor of ActRII receptors or an activin-ActRII inhibitor. Inhibitors of ActRII receptors include ActRIIA inhibitors and ActRIIB inhibitors. Inhibitors of ActRII receptors can be polypeptides comprising activin-binding domains of ActRII. In certain embodiments, the activin-binding domain comprising polypeptides are linked to an Fc portion of an antibody (*i.e.,* a conjugate comprising an activin-binding domain comprising polypeptide of an ActRII receptor and an Fc portion of an antibody is generated). In certain embodiments, the activin-binding domain is linked to an Fc portion of an antibody via a linker, *e.g.,* a peptide linker.

An exemplary activin-binding ActRIIA polypeptide fused to a human Fc domain is provided in SEQ ID NO: 1.

SEQ ID NO:1

ILGRSETQECLFFNANWEKDRTNQTGVEPCYGDKDKRRHCFATWKNISGS
IEIVKQGCWLDDINCYDRTDCVEKKDSPEVYFCCCEGNMCNEKFSYFPEM

EVTQPTSNPVTPKPPTGGGTHTCPPCPAPELLGGPSVFLFPPKPKDTLMI
SRTPEVTCVVVDVSHEDPEVKFNWYVDGVEVHNAKTKPREEQYNSTYRVV
SVLTVLHQDWLNGKEYKCKVSNKALPVPIEKTISKAKGQPREPQVYTLPP
SREEMTKNQVSLTCLVKGFYPSDIAVEWESNGQPENNYKTTPPVLDSDGS
FFLYSKLTVDKSRWQQGNVFSCSVMHEALHNHYTQKSLSLSPGK

An exemplary fusion protein comprising a soluble extracellular domain of ActRIIB fused to an Fc domain is provided in SEQ ID NO: 2.

SEQ ID NO:2

ETRECIYYNANWELERTNQSGLERCEGEQDKRLHCYASWRNSSGTIELVK
KGCWDDDFNCYDRQECVATEENPQVYFCCCEGNFCNERFTHLPEAGGPEV
TYEPPPTGGGTHTCPPCPAPELLGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTV
DKSRWQQGNVFSCSVMHEALHNHYTQKSLSLSPGK

Further examples of non-antibody proteins selected for activin or ActRIIA binding and methods for design and selection of the same are found in WO/2002/088171, WO/2006/055689, WO/2002/032925, WO/2005/037989, US 2003/0133939, and US 2005/0238646, each of which is incorporated herein by reference in its entirety.

Any combination of the above therapeutic agents, suitable for treatment of the diseases or symptoms thereof, can be administered. Such therapeutic agents can be administered in any combination with Compound I or a pharmaceutically acceptable salt, solvate, hydrate, stereoisomer, tautomer or racemic mixtures thereof, at the same time or as a separate course of treatment.

## 7.5 CYCLING THERAPY

In certain embodiments, Compound I or a pharmaceutically acceptable salt, solvate, hydrate, stereoisomer, tautomer or racemic mixtures thereof provided herein is cyclically administered to a patient. Cycling therapy involves the administration of an active agent for a period of time, followed by a rest (*i.e.,* discontinuation of the administration) for a period of time, and repeating this sequential administration. Cycling therapy can reduce the development of resistance to one or more of the therapies, avoid or reduce the side effects of one of the therapies, and/or improve the efficacy of the treatment.

Consequently, in one embodiment, a compound provided herein is administered daily in a single or divided doses in a four to six week cycle with a rest period of about a week or two weeks. Cycling therapy further allows the frequency, number, and length of dosing cycles to be increased. Thus, another embodiment encompasses the administration of a compound provided herein for more cycles than are typical when it is administered alone. In yet another embodiment, a compound provided herein is administered for a greater number of cycles than would typically cause dose-limiting toxicity in a patient to whom a second active ingredient is not also being administered.

In one embodiment, a compound provided herein is administered daily and continuously for three or four weeks at a dose of from about 0.1 mg to about 500 mg per day, followed by a rest of one or two weeks. In other embodiments, the dose can be from about 1 mg to about 300 mg, from about 0.1 mg to about 150 mg, from about 1 mg to about 200 mg, from about 10 mg to about 100 mg, from about 0.1 mg to about 50 mg, from about 1 mg to about 50 mg, from about 10 mg to about 50 mg, from about 20 mg to about 30 mg, or from about 1 mg to about 20 mg, followed by a rest.

In one embodiment, a compound provided herein and a second active ingredient are administered orally, with administration of the compound provided herein occurring 30 to 60 minutes prior to the second active ingredient, during a cycle of four to six weeks. In another embodiment, the combination of a compound provided herein and a second active ingredient is administered by intravenous infusion over about 90 minutes every cycle.

Typically, the number of cycles during which the combination treatment is administered to a patient will be from about one to about 24 cycles, from about two to about 16 cycles, or from about four to about three cycles.

## 7.6 PHARMACEUTICAL COMPOSITIONS AND DOSAGE FORMS

Pharmaceutical compositions can be used in the preparation of individual, single unit dosage forms. Pharmaceutical compositions and dosage forms provided herein comprise a compound provided herein, or a pharmaceutically acceptable salt, solvate, hydrate, stereoisomer, racemate, clathrate, or prodrug thereof. Pharmaceutical compositions and dosage forms can further comprise one or more excipients.

Pharmaceutical compositions and dosage forms provided herein can also comprise one or more additional active ingredients. Examples of optional second, or additional, active ingredients are disclosed above.

Single unit dosage forms provided herein are suitable for oral, mucosal (*e.g.*, nasal, sublingual, vaginal, buccal, or rectal), parenteral (*e.g.*, subcutaneous, intravenous, bolus injection, intramuscular, or intraarterial), topical *(e.g.,* eye drops or other ophthalmic preparations), transdermal or transcutaneous administration to a patient. Examples of dosage forms include, but are not limited to: tablets; caplets; capsules, such as soft elastic gelatin capsules; cachets; troches; lozenges; dispersions; suppositories; powders; aerosols (*e.g.*, nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; eye drops or other ophthalmic preparations suitable for topical administration; and sterile solids (*e.g.*, crystalline or amorphous solids) that can be reconstituted to provide liquid dosage forms suitable for parenteral administration to a patient.

The composition, shape, and type of dosage forms will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms are used will vary from one another will be readily apparent to those skilled in the art. *See, e.g.,* Remington's Pharmaceutical Sciences, 20th ed., Mack Publishing, Easton PA (2000).

In one embodiment, pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form

will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, provided are pharmaceutical compositions and dosage forms that contain little, if any, lactose other mono- or di-saccharides. As used herein, the term "lactose-free" means that the amount of lactose present, if any, is insufficient to substantially increase the degradation rate of an active ingredient.

Lactose-free compositions can comprise excipients that are well known in the art and are listed, for example, in the *U.S. Pharmacopeia* (USP) 25-NF20 (2002). In general, lactose-free compositions comprise active ingredients, a binder/filler, and a lubricant in pharmaceutically compatible and pharmaceutically acceptable amounts. In one embodiment, lactose-free dosage forms comprise active ingredients, microcrystalline cellulose, pre-gelatinized starch, and magnesium stearate.

Also provided are anhydrous pharmaceutical compositions and dosage forms comprising active ingredients, since water can facilitate the degradation of some compounds. For example, the addition of water (*e.g.,* 5%) is widely accepted in the pharmaceutical arts as a means of simulating long-term storage in order to determine characteristics such as shelf-life or the stability of formulations over time. *See, e.g.,* Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms can be prepared using anhydrous or low moisture containing ingredients and low moisture or low humidity conditions. Pharmaceutical compositions and dosage forms that comprise lactose and at least one active ingredient that comprises a primary or secondary amine are anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are, in one embodiment, packaged using materials known to prevent exposure to water such that they can be included in suitable formulary kits. Examples of suitable packaging include, but are not limited to, hermetically sealed foils, plastics, unit dose containers (*e.g.,* vials), blister packs, and strip packs.

Also provided are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. In one embodiment, dosage forms comprise a compound provided herein in an amount of from about 0.10 to about 500 mg. In other embodiments, dosage forms comprise a compound provided herein in an amount of about 0.1, 1, 2, 5, 7.5, 10, 12.5, 15, 17.5, 20, 25, 50, 100, 150, 200, 250, 300, 350, 400, 450, or 500 mg.

In other embodiments, dosage forms comprise the second active ingredient in an amount of 1 to about 1000 mg, from about 5 to about 500 mg, from about 10 to about 350 mg, or from about 50 to about 200 mg. Of course, the specific amount of the second active agent will depend on the specific agent used, the diseases or disorders being treated or managed, and the amount(s) of a compound provided herein, and any optional additional active agents concurrently administered to the patient.

**Oral Dosage Forms**

Pharmaceutical compositions that are suitable for oral administration can be provided as discrete dosage forms, such as, but not limited to, tablets (*e.g.*, chewable tablets), caplets, capsules, and liquids (*e.g.*, flavored syrups). Such dosage forms contain predetermined amounts of active ingredients, and may be prepared by methods of pharmacy well known to those skilled in the art. *See generally,* Remington's Pharmaceutical Sciences, 20th ed., Mack Publishing, Easton PA (2000).

Oral dosage forms provided herein are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g.*, powders, tablets, capsules, and caplets) include, but are not limited to, starches, sugars, micro-crystalline cellulose, diluents, granulating agents, lubricants, binders, and disintegrating agents.

In one embodiment, oral dosage forms are tablets or capsules, in which case solid excipients are employed. In another embodiment, tablets can be coated by standard aqueous or nonaqueous techniques. Such dosage forms

can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms provided herein include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g.*, ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g.,* Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Examples of fillers suitable for use in the pharmaceutical compositions and dosage forms provided herein include, but are not limited to, talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions is, in one embodiment, present in from about 50 to about 99 weight percent of the pharmaceutical composition or dosage form.

Disintegrants may be used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients may be used to form solid oral dosage forms. The amount of disintegrant used varies based upon the type of formulation, and is readily discernible to those of ordinary skill in the art. In one embodiment, pharmaceutical compositions comprise from about 0.5 to about 15 weight percent of disintegrant, or from about 1 to about 5 weight percent of disintegrant.

Disintegrants that can be used in pharmaceutical compositions and dosage forms include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.,* peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants may be used in an amount of less than about 1 weight percent of the pharmaceutical compositions or dosage forms into which they are incorporated.

In one embodiment, a solid oral dosage form comprises a compound provided herein, anhydrous lactose, microcrystalline cellulose, polyvinylpyrrolidone, stearic acid, colloidal anhydrous silica, and gelatin.

**Controlled Release Dosage Forms**

Active ingredients such as the compounds provided herein can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; 6,699,500 each of which is incorporated herein by reference. Such dosage forms can be used to provide slow or controlled release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions.

Suitable controlled release formulations known to those of ordinary skill in the art, including those described herein, can be readily selected for use with the active ingredients provided herein. Thus, the compositions provided encompass single unit dosage forms suitable for oral administration such as, but not limited to, tablets, capsules, gelcaps, and caplets that are adapted for controlled release.

All controlled release pharmaceutical products have a common goal of improving drug therapy over that achieved by their non controlled counterparts. Ideally, the use of an optimally designed controlled release preparation in medical treatment is characterized by a minimum of drug substance being employed to cure or control the condition in a minimum amount of time. Advantages of controlled release formulations include extended activity of the drug, reduced dosage frequency, and increased subject compliance. In addition, controlled release formulations can be used to affect the time of onset of action or other characteristics, such as blood levels of the drug, and can thus affect the occurrence of side (e.g., adverse) effects.

Most controlled release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled release of an active ingredient can be stimulated by various conditions including, but not limited to, pH, temperature, enzymes, water, or other physiological conditions or compounds.

In certain embodiments, the drug may be administered using intravenous infusion, an implantable osmotic pump, a transdermal patch, liposomes, or other modes of administration. In one embodiment, a pump may be used (*see,* Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used. In yet another embodiment, a controlled release system can be placed in a subject at an appropriate site determined by a practitioner of skill, i.e., thus requiring only a fraction of the systemic dose (*see, e.g.,* Goodson, Medical Applications of Controlled Release, vol. 2, pp. 115-138 (1984)). Other controlled release systems are discussed in the review by Langer (Science 249:1527-1533 (1990)). The active ingredient can be dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient in such parenteral compositions is highly dependent on the specific nature thereof, as well as the needs of the subject.

### Parenteral Dosage Forms

Parenteral dosage forms can be administered to patients by various routes including, but not limited to, subcutaneous, intravenous (including bolus injection), intramuscular, and intraarterial. In some embodiments, administration of a parenteral dosage form bypasses patients' natural defenses against contaminants, and thus, in these embodiments, parenteral dosage forms are sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms. For example, cyclodextrin and its derivatives can be used to increase the solubility of a compound provided herein. *See, e.g.,* U.S. Patent No. 5,134,127, which is incorporated herein by reference.

**Topical and Mucosal Dosage Forms**

Topical and mucosal dosage forms provided herein include, but are not limited to, sprays, aerosols, solutions, emulsions, suspensions, eye drops or other ophthalmic preparations, or other forms known to one of skill in the art. *See, e.g.,* Remington's Pharmaceutical Sciences, 16th, 18th and 20th eds., Mack Publishing, Easton PA (1980, 1990 and 2000); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels.

Suitable excipients (*e.g*., carriers and diluents) and other materials that can be used to provide topical and mucosal dosage forms encompassed herein are well known to those skilled in the pharmaceutical arts, and depend on the particular tissue to which a given pharmaceutical composition or dosage form will be applied. In one embodiment, excipients include, but are not limited to, water, acetone, ethanol, ethylene glycol, propylene glycol, butane-1,3-diol, isopropyl myristate, isopropyl palmitate, mineral oil, and mixtures thereof to form solutions, emulsions or gels, which are nontoxic and pharmaceutically acceptable. Moisturizers or humectants can also be added to pharmaceutical compositions and dosage forms. Examples of additional ingredients are well known in the art. *See, e.g.,* Remington's Pharmaceutical Sciences, 16th,18th and 20th eds., Mack Publishing, Easton PA (1980, 1990 and 2000).

The pH of a pharmaceutical composition or dosage form may also be adjusted to improve delivery of one or more active ingredients. Also, the polarity of a solvent carrier, its ionic strength, or tonicity can be adjusted to improve delivery. Compounds such as stearates can also be added to pharmaceutical compositions or dosage forms to alter the hydrophilicity or lipophilicity of one or more active ingredients so as to improve delivery. In other embodiments, stearates can serve as a lipid vehicle for the formulation, as an emulsifying agent or surfactant, or as a delivery-enhancing or penetration-enhancing agent. In other embodiments, salts, solvates, hydrates, prodrugs, clathrates, or stereoisomers of the active ingredients can be used to further adjust the properties of the resulting composition.

**KITS**

In one embodiment, active ingredients provided herein are not administered to a patient at the same time or by the same route of administration. In another embodiment, provided are kits which can simplify the administration of appropriate amounts of active ingredients.

In one embodiment, a kit comprises a dosage form of a compound provided herein. Kits can further comprise additional active ingredients such as other anti-inflammatory, immunomodulatory or immunosuppressant compounds, or a combination thereof. Examples of the additional active ingredients include, but are not limited to, those disclosed herein.

In other embodiments, kits can further comprise devices that are used to administer the active ingredients. Examples of such devices include, but are not limited to, syringes, drip bags, patches, and inhalers.

Kits can further comprise cells or blood for transplantation as well as pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

## 8. EXAMPLES

The following Examples are presented by way of illustration, not limitation.

### 8.1 EXAMPLE 1: EFFECT OF TEST COMPOUND ON CYTOKINE AND CHEMOKINE PRODUCTION IN ANTI-HUMAN CD3-STIMULATED HUMAN T CELLS

This example demonstrates the effect of 3-(5-amino-2-methyl-4-oxoquinazolin-3(4*H*)-yl)piperidine-2,6-dione (test compound) on cytokine and chemokine production in anti-human CD3-stimulated human T cells using multiplex Luminex Technology.

The following abbreviations are used:

| Abbreviation | Explanation or Definition |
|---|---|
| IL | Interleukin |

| | |
|---|---|
| G-CSF | Granulocyte Colony Stimulating Factor |
| GM-CSF | Granulocyte Macrophage Colony Stimulating Factor |
| IFN-γ | Interferon Gamma |
| TNF-α | Tumor Necrosis Factor Alpha |
| RANTES | Regulated on Activation, Normal T Cell Expressed and Secreted |

The following materials were used in this study:

RPMI-1640 Media supplemented with 10% FBS, 100 units/mL penicillin, 100 mg/mL streptomycin and 2 mM L-glutamine (Life Technologies)
RosetteSep® Human T- Cell Enrichment Cocktail (StemCell, Cat# 15061)
Luminex Human Cytokine/Chemokine 12-Plex Kit (Millipore, Cat# MPXHCYTO-60K-12)
Luminex IS100 instrument (Millipore)
Anti-human CD3 antibody, OKT3 clone (eBioscience, Cat# 16-0037-85)

The test compounds were prepared as stock solutions of 4 mM in DMSO. T cells were isolated from buffy coat by negative selection using the RosetteSep® T Cell Enrichment Cocktail according to manufacturer's procedures.

All 96-well plates were pre-coated with 3 μg/mL anti-human CD3 antibody in 100 μL 1X PBS for 4 hours at 37°C. The plates were washed 3 times with RPMI-1640 Complete Media prior to the T cell assay. The T cells were then plated in anti-CD3-pre-coated plates at a density of 2.5 x $10^5$ cells/well in 180 μL RPMI-1640 Complete Media. The cells were treated with 20 μL 10X titrated test compound at 10, 1, 0.1, 0.01, 0.001, 0.0001, and 0.00001 μM in duplicate. The final DMSO concentrations were 0.25%. The plates were incubated for 48 hours at 37°C, 5% $CO_2$.
After 48 hours, the supernatants were harvested and tested by a multiplex cytometric bead array (CBA) assay for the following cytokines/chemokines: IL-2, IL-3, IL-5, IL-10, IL-13, IL-15, IL-17A, GM-CSF, G-CSF, IFN-γ, TNF-α, and RANTES.
The CBA plates were analyzed on the Luminex IS 100 instrument.

Data from each donor was graphed using GraphPad Prism 5.0 software and expressed as mean pg/mL ± SEM and % of DMSO control ± SEM.

The test compound demonstrated immunomodulatory activity in anti-CD3 stimulated primary human T cells, altering the production of several cytokines and chemokines. Baseline levels of cytokines and chemokines produced by stimulated human T cells incubated with vehicle are presented in Table 1 below.

Table 1: Baseline levels of cytokines and chemokines

| Cytokine/Chemokine | Baseline Amount Produced (pg/mL) |
|---|---|
| IL-2 | 31 |
| IL- | 38 |
| IL-5 | 27 |
| IL-10 | 449 |
| IL-13 | 205 |
| IL-17A | 19 |
| GM-CSF | 132 |
| IFN-γ | 1271 |
| TNF-α | 411 |
| RANTES | 314 |

The test compound enhanced IL-2, IL-3, IL-5, IL-10, IL-13, GM-CSF, IFN-γ, RANTES, and TNF-α production in stimulated human T cells. The enhancement of production by the test compound was largely concentration-dependent for most of the cytokines and chemokines, except for IL-10 and IL-5. The test compound enhanced IL-10 production at lower concentrations but inhibited enhancement of IL-10 production at higher concentrations. The test compound increased IL-5 production 3- and 4-fold at 0.01 and 0.1 μM, respectively, showing less enhancement at both lower and higher concentrations. The effect of the test compound on cytokine and chemokine production in anti-CD3-stimulated human T cells, expressed as absolute amount produced and as percentage of vehicle control cells are provided in FIGs. 1 and 2, respectively. The dashed line denotes the level equivalent to double the baseline production ($EC_{200}$) in FIG. 2.

## 8.2 EXAMPLE 2: ANTI-INFLAMMATORY ACTIVITY

Anti-inflammatory activity of 3-(5-amino-2-methyl-4-oxoquinazolin-3(4*H*)-yl)piperidine-2,6-dione (test compound) was studied in human peripheral blood mononuclear cells (hPBMC). Luminex Technology was used to determine the inhibitory (enhancement) concentration, $IC_{50}$ for the compound for the simultaneous profiling of pro-inflammatory cytokines/chemokines and IL-10 (anti-inflammatory cytokine) from LPS-stimulated healthy human donor PBMCs.

The following abbreviations are used:

| Abbreviation | Explanation or Definition |
|---|---|
| GM-CSF | Granulocyte Macrophage Colony Stimulating Factor |
| IL | Interleukin |
| LPS | lipopolysaccharide |
| MCP-1 | monocyte chemotactic protein-1 |
| MDC | Macrophage-derived chemokine |
| MIP-1α | Macrophage inflammatory protein-1alpha |
| MIP-1β | Macrophage inflammatory protein-1beta |
| PBMC | Peripheral Blood Mononuclear cells |
| PPM | Pleiotrophic Pathway Modifier |
| RANTES | Regulated upon Activation Normal T-cell Expressed, and Secreted |
| TNF-α | Tumor Necrosis Factor- Alpha |

50 ml Buffy coat from healthy donors was obtained from Blood Center of New Jersey (East Orange, New Jersey). Lipopolysaccharide (strain)(Cat# L-1887) was purchased from Sigma. Milliplex kits with antibody bound beads for Luminex xMAP Technology was purchased from Millipore (Billerica, Massachusetts) and combined into multiplex format prior to assay.

### Purification of Human Peripheral Blood Mononuclear Cells

50 ml human buffy coat was aliquoted 25 ml each into two 50 ml conical tubes and 25 ml sterile HBSS was added to each conical tube. The tubes were gently mixed by inverting. Fifteen ml of room temperature Ficoll-Paque Plus (GE Healthcare (location); cat# 17-1440-02) was aliquoted into four 50 ml conical tubes. Then 25 ml of the Buffy coat/HBSS mixture was layered gently and slowly on top of the Ficoll. The samples were centrifuged at 450 rpm for 35 minutes. The top layered containing plasma was pipetted off and discarded. The interface containing mononuclear cells was transferred into two 50 ml conical tubes. Both conical tubes were filled to total volume of 50 ml with HBSS and centrifuged at 1200 rpm for 10 minutes. The cells were washed again in HBSS and spun at 1000 rpm for 10 minutes. Cell pellet was resuspended with 20 ml RPMI complete medium (RPMI/5% human sera/1x pen/strep/glut) and counted.

### Treatment of Human Peripheral Blood Mononuclear Cells

One hundred μl (2x106/ml) of hPBMCs were added to each well of a 96 well flat-bottom plate (final cell count = 2x105/well) and incubated at 37 °C for 1 hour. Twenty μl (10x) compound was added to each test well and twenty μl medium containing 2.5% DMSO was added to each control well ([DMSO]final=0.25%) and plate was incubated for 1 hour at 37 °C. Cells were then stimulated with 80 μl of 2.5 ng/ml LPS ([LPS]final=1 ng/ml) and incubated for 18 hours at 37 °C.

50 μl supernatant from each well was transferred into 3 new round-bottomed 96 well plates and stored at -20 °C for Luminex analysis. Duplicate wells were performed for each sample.

### Luminex Analysis

Supernatant samples were analyzed for cytokines in multiplex format according to the manufacturer's instructions (Millipore, Billerica, Ma 01821) using a Luminex IS100 instrument. IL-12 and GM-CSF analyses were done in a two-plex format using neat supernatants while all other cytokines were done in a multiplex format using supernatants diluted 1:20. Data analysis was performed using Upstate Beadview software. $IC_{50}$s were calculated using non-linear regression, sigmoidal dose-response, constraining the top to 100% and bottom to 0%, allowing variable slope. The $EC_{50}$s were based on the upper constraint of the sigmoidal curves equaling 246.9%, representing the average IL-10 enhancement produced by pomalidomide (control) at 10 μM
and the lower constraint to 100%. The $IC_{50}$ were performed using GraphPad Prism v5.00. The data values represent the mean + SEM (standard error of the mean) of n (number of experiments in duplicate).

As demonstrated by data in Table 2 below and FIG. 3, the test compound has varied potencies for the inhibitions of the multiple cytokines examined, e.g., Il-6, IL-8, IL-1β, GM-CSF, MDC, MIP-1α, MIP-1β, and TNF-α, in general. Also, the test compound enhanced production of IL-10, MCP-1, and RANTES with various potencies as provided in Table 3 and FIG. 4.

Table 2: Summary of Cytokine Inhibitory Profile of Test Compound

| Cytokine | Test compound $IC_{50}$ (μM) |
|---|---|
| IL-6 | 0.060 |
| IL-8 | >10 |
| IL-1β | 0.054 |
| GM-CSF | 0.95 |
| MDC | 0.062 |
| MIP-1α | 0.30 |
| MIP-1β | >10 |
| TNF-α | 0.034 |

Table 3: Cytokine Profile Summary of - mean % of control at 0.1 μM

| Cytokine | Test compound (% of control) |
|---|---|
| IL-10 | 480 |
| MCP-1 | 236 |
| RANTES | 131 |

### 8.3 EXAMPLE 3: EFFECT ON HUMAN NATURAL KILLER (NK) CELL FUNCTION IN RESPONSE TO IGG/RITUXIMAB

In this example, the capacity of the test compound to enhance human NK cell function in response to IgG/Rituximab was studied. The immunomodulatory activity of the test compound was compared in two assays of natural killer (NK) cell functions (1) IgG- and IL-2-induced interferon-gamma (IFN-γ) production and (2) killing activity, as measured in an *in vitro* ADCC (antibody-dependent cellular cytotoxity) model.

The following abbreviations are used:

| Abbreviation | Explanation or Definition |
|---|---|
| ABC-DLBCL | Activated B Cell-like Diffuse Large B Cell Lymphoma |
| ADCC | Antibody Dependent Cellular Cytoxicity |
| DMSO | Dimethyl sulfoxide |
| IgG | Immunoglobulin G |
| IFN-γ | Interferon-gamma |
| NK | Natural killer |
| PPM | Pleiotropic Pathway Modifier |
| rhIL-2 | Human Recombinant Interleukin-2 |

The materials used in the study and their sources are provided below:

Buffy Coat from healthy volunteers (Blood Center of New Jersey)
Ficoll-Hypaque Plus (Fisher Scientific Co LLC, PA, Cat # 17144002)
RPMI-1640 Medium supplemented with 10% FBS (fetal bovine serum), 100 units/mL penicillin, 100 mg/mL streptomycin, and 2 mM L-glutamine (Invitrogen, Cat # 21870-076)
RPMI-1640 Medium (without phenol red) supplemented with 10% FBS, 100 units/mL penicillin, 100 mg/mL streptomycin, and 2 mM L-glutamine (Invitrogen, Cat # 11835-030)
Rituximab (Rituxan, Roche, Inc.) (Cat No. DIN 02241927, Lot No. B50177)

Human AB+ serum (Gemini Bio Products, CA, Cat # 100-512)
CytoTox 96 Non-Radioactive Cytotoxicity Assay Kit (Promega, WI, Cat # G1780)
RosetteSep Human NK Cell Enrichment Cocktail (Stem Cell Technologies, Vancouver, BC, Cat# 15065)
Mouse anti-human CD56+ conjugated APC (BD Biosciences, CA, Cat # 555518)
Human Immunoglobulin G from Serum (IgG) (Sigma, St. Louis, MO; Cat # I2511-10MG)
Human Recombinant IL-2 (R&D Systems, MN, Cat # 202-IL-050/CF)
Human IFN-gamma ELISA Kit (ThermoFisher, Cat # PIEHIFNG5)

The following cell lines were used:

Activated B cell-like - diffuse large B cell lymphoma (ABC-DLBCL): Riva cells (NCI, MD)
Germinal center B-cell-like - diffuse large B cell lymphoma (GCB-DLBCL): WSU-DLCL2 (Celgene Signal, CA)
Farage (ATCC, VA)
Follicular lymphoma: DoHH2 (DSMZ, Germany)
Burkitt's lymphoma (BL): Raji (ATCC, VA).

NK cells from healthy donors were isolated from buffy coat blood by negative selection using the RosetteSep NK cell enrichment cocktail (Stem Cell Technologies, Vancouver, BC) prior to Ficoll-Hypaque (Fisher Scientific Co LLC, PA) density gradient centrifugation following the manufacturers' instructions. CD56+ NK cells were isolated to ~85% purity, as determined by flow cytometry (BD Biosciences, CA).

**NK IgG-induced Interferon-Gamma (IFN-Gamma) Assay**

Ninety-six-well flat-bottom plates were coated with 100 μg/mL of human IgG (Sigma) overnight at 4°C. The next day, unbound IgG was washed away with cold 1X PBS. NK cells were then plated in the IgG-coated 96-well plates at 2 x 105 cells per well in 180 μL RPMI-1640 Media and 10 ng/mL of rhIL-2 (R & D Systems, MN) was added. The test compound was added in a volume of 20 μL DMSO. Final concentrations of the test compound were 0.0001, 0.001, 0.01, 0.1, 1, or 10 μM. Final DMSO concentrations were 0.25%. After 48 hours, the supernatants were harvested and analyzed by ELISA for IFN-γ production.

Data used to determine the ability of the test compound to enhance NK cell IFN-γ production in response to immobilized IgG and rhIL-2 stimulation was analyzed for each donor using GraphPad Prism v5.0 software. The data are presented in two ways, (1) as the absolute amount if IFN-γ produced (pg/mL ± SEM) and (2) as the percentage of the amount of IFN-γ produced in the presence of 1 μM pomalidomide. The $EC_{50}$ is the concentration of the test compound providing half-maximal IFN-γ production, with maximal production defined as the amount of IFN-γ produced in the presence of 1 μM pomalidomide. $EC_{50}$ values were calculated using non-linear regression, sigmoidaldose-response constraining the top to 100% and bottom to 0% allowing for a variable slope. $EC_{50}$ for the test compound was 0.0015 μM.

The test compound enhanced NK cell IFN-γ production in a dose dependent manner in response to immobilized IgG and IL-2 stimulation. Results are provided in FIG. 5 (expressed as pg/mL of IFN-γ produced), respectively. FIG. 6 provides results expressed as a percentage of increased IFN-γ produced relative to the IFN-γ produced in the presence of pomalidomide at 1 μM for the test compound. Each value plotted in FIGs. 5 and 6 represents the mean of 12-14 determinations ± SEM.

**ADCC Assay**

Purified NK cells (5 x 104) were seeded in 96-well U-bottom plates in 100 μL of RPMI-1640 medium without phenol (Invitrogen) + 2% human AB+ serum (Gemini Bio Products, CA) and treated with 10 ng/mL rhIL-2 and rituximab (5 μg/mL) plus different concentrations of the test compound at 0.01 to 10 μM for 48 hours.

Various lymphoma cell lines (GCB-DLBCL: WSU-DLCL2 and Farage; Follicular lymphoma:DoHH2; ABC-DLBCL: Riva; Burkitt's lymphoma [BL]: Raji) were treated with 5 μg/mL rituximab for 30 minutes at 37°C. Unbound rituximab was washed off, target cells (5 x 103/100 μL/well) were added to the pretreated effector cells (NK cells) at a 10:1 ratio, and the two were co-incubated for 4 hours at 37°C. Control conditions consisted of NK cells plus tumor cells treated with (1) medium alone, (2) rituximab only, or (3) IL-2 alone. Using an aliquot of supernatant (50 μL), NK cell cytotoxicity against tumor cells was analyzed using a standard lactate dehydrogenease (LDH) release assay to measure ADCC (CytoTox 96 Non-Radioactive Cytoxicity Assay, Promega, WI). Spontaneous release by target cells alone was < 15% of the maximum release, as determined with target cells lysed in 1% Triton X-100. The experimental release was corrected by subtraction of the spontaneous release of effector cells at the corresponding dilution. The percentage of specific lysis was calculated according to the formula:

Percentage specific lysis = 100 x (experimental – effector spontaneous – target spontaneous) / (target maximum – target spontaneous).

The test compound induced dose-dependent NK cell-mediated ADCC in all cell lines. Three experiments were conducted for each cell line and samples from each of three donors were tested in each experiment. Data are presented in FIG. 8, as mean of 9 determinations ± SEM.

**8.4 EXAMPLE 4: EFFECT ON THE EXPRESSION OF TRANSCRIPTION FACTORS IN PRIMARY HUMAN B CELL DIFFERENTIATION MODEL**

In this example, the effect of 3-(5-amino-2-methyl-4-oxoquinazolin-3(4*H*)-yl)piperidine-2,6-dione (test compound) on the expression of transcription factors controlling plasma cell differentiation, and immunoglobulin production, using an *in vitro* human B-cell differentiation culture system.

The following abbreviations are used in this example:

| Abbreviation or Specialist Term | Explanation or Definition |
|---|---|
| BCL6 | B-cell lymphoma 6 protein |
| BLIMP-1 | B-lymphocyte-induced maturation protein 1 |
| EtOH | Ethanol |
| FBS | Fetal bovine serum |
| DMSO | Dimethyl sulfoxide |
| IRF-4 | Interferon regulatory factor 4 |
| MFI | Mean fluorescence intensity |
| PAX5 | Paired box protein Pax-5 |
| SLE | Systemic lupus erythematosus |
| XBP-1 | X-box binding protein 1 |

50 ml Buffy coat from healthy donors were obtained from Blood Center of New Jersey. SLE Lupus PBMC samples were obtained from Conversant Bio (Huntsville, Alabama 35806).

The following cell culture reagents were used in this study.

| ITEM | Source |
|---|---|
| Iscoves Modified Dulbecco medium | Invitrogen |
| Fetal Bovine Serum | Lonza |
| Human Insulin | Sigma |
| Human Transferrin | Sigma |
| penicillin/ streptomycin | Lonza |
| Recombinant Human IL-2 | R & D Systems |
| Recombinant Human IL-6 | R & D Systems |
| Recombinant Human IL-10 | R & D Systems |
| Recombinant Human IL-15 | R & D Systems |
| CD40 Ligand/TNFSF5/ histidine-tagged | R & D Systems |
| polyHistidine mouse IgG1 antibody | R & D Systems |
| ODN 2006- Human TLR9 ligand | Invivogen |

(continued)

| ITEM | Source |
|---|---|
| Human Interferon ALPHA A | PB interferon source |

The following were used in flow cytometry analysis.

| ITEM | Source |
|---|---|
| FITC anti-human CD19 | IBD Pharmigen |
| FITC anti-human CD20 | IBD Pharmigen |
| PE anti-human CD27 | IBD Pharmigen |
| PE anti-human CD38 | IBD Pharmigen |
| APC anti-human CD38 | IBD Pharmigen |
| FITC anti-mouse IgG1k Isotype | IBD Pharmigen |
| PE anti-mouse IgG1k Isotype | IBD Pharmigen |
| FITC anti-mouse IgG2bk Isotype | IBD Pharmigen |
| APC anti-mouse IgG1k Isotype | IBD Pharmigen |
| Stain Buffer | IBD Pharmigen |

The following gene primers were used for RT-PCR:

| ITEM | Source |
|---|---|
| AICDA gene expression assay | Applied Biosystem |
| BCL6 gene expression assay | Applied Biosystem |
| GAPDH gene expression assay | Applied Biosystem |
| IGJ gene expression assay | Applied Biosystem |
| IRF4 gene expression assay | Applied Biosystem |
| PAX5 gene expression assay | Applied Biosystem |
| PRDM1 gene expression assay | Applied Biosystem |
| XBP1 gene expression assay | Applied Biosystem |
| Reverse Transcription Kit | Applied Biosystem |
| Master Mix | Applied Biosystem |

### 8.4.1 Purification of hPBMCs

Fifty ml human buffy coat was aliquoted 25 ml each into two 50 ml conical tubes and 25 ml sterile HBSS was added to each conical tube. The tubes were gently mixed by inverting. Fifteen ml of room temperature Ficoll-Paque Plus (GE Healthcare; cat# 17-1440-02) was aliquoted into four 50 ml conical tubes. Then 25 ml of the Buffy coat/HBSS mixture was layered gently and slowly on top of the Ficoll. The samples were centrifuged at 450 rpm for 35 minutes. The top layered containing plasma was pipetted off and discarded. The interface containing mononuclear cells was transferred into two 50 ml conical tubes. Both conical tubes were filled to total volume of 50 ml with HBSS and centrifuged at 1200 rpm for 10 minutes. The cells were washed again in HBSS and spun at 1000 rpm for 10 minutes. Cell pellet was resuspended with 20 mL of B cell media (Iscoves +10% PFBS, 1% P/S, and 5 μg/mL human insulin) and counted on the cell counter.

#### 8.4.2 B Cell Enrichment CD19+

Purified PBMCs were counted and aliquoted at $2 \times 10^8$ cells per tube. The cells were centrifuged at 1200 rpm for 5 minutes and then supernatants were discarded. The cells were resuspended in 4 mL of Robosep Buffer (Stemcell Technologies catalog # 20104) and transferred to a 14 mL polystyrene round bottom tube (BD catalog # 352057) and mixed well. Then 200 μL of EasySep Human B cell enrichment cocktail was added (StemCell Technologies catalog # 19054). Samples were vortexed and incubated at room temperature for 10 minutes. Next 300 μL of EasySep Magnetic particles (vortexed) (StemCell Technologies catalog # 19054) were added to the tube. Samples were vortexed and incubated at room temperature for 5 minutes. After the 5 minute incubation, 5 mL of Robosep buffer was added to the tube and mixed well by pipetting up and down. The tube was immediately places in the silver magnet (StemCell Technologies catalog # 19054) and incubated at room temperature for 5 minutes. After incubation, in one continuous motion, invert magnet and tube and pour off desired fraction into a 50 mL conical.. These procedures were repeated for remaining PBMCs (per one donor) and combined. The combined fraction was centrifuged at 1200 rpm for 5 minutes and then supernatants were discarded and cells were resuspended in 5 mL of B cell media. The isolated CD 19+ cells were counted on the cell counter.

#### 8.4.3 B cell Differentiation Assay

Step 1 -B cell Activation- day 0 through day 4: Prepare fresh B cell cocktail by adding 50 μg/mL of human transferrin to B cell media. (Iscoves +10% PFBS, 1% P/S, and 5 μg/mL human insulin). Filter required volume of media needed for experiment through a 0.22 μM filter. Add B cell differentiation cocktail (final concentration): recombinant human IL-2 (20U/mL), IL-10 (50 ng/mL), IL-15 (10 ng/mL), CD40 Ligand/TNFSF5/ histidine-tagged (50 ng/mL), polyHistidine mouse IgG1 antibody (5 μg/mL), and ODN 2006- Human TLR9 ligand (10 μg/mL) to cells. Five milliliters ($1 \times 10^5$/ml) of CD19+ B cell were added to each well of a 6 well flat-bottom plate (final cell count = $5 \times 10^5$/well). Five μL (1x) ± compound/DMSO was added to each test well (0.1% final DMSO) and incubated at 37°C for 4 days.

Step 2 -Plasmablast Generation- day 4 through day 7: Cells were harvested and counted on the cell counter; an aliquot was removed for flow analysis, the remaining cells were washed with PBS. Prepare fresh B cell cocktail by adding 1μg/ml of human transferrin to B cell media. (Iscoves +10% PFBS, 1% P/S, and 5 μg/mL human insulin). Filter required volume of media needed for experiment through a 0.22 μM filter. Add B cell differentiation cocktail (final concentration): recombinant human IL-2 (20U/mL), IL-10 (50 ng/mL), IL-15 (10 ng/mL), IL-6 (50ng/mL) to cells. Add fresh B cell cocktail and transfer cells back to the original wells and bring volume back to 5 mL. Five μL (1x) ± compound/DMSO was added to each test well (0.1% final DMSO) and incubated at 37°C for 4 days.

On day 7, cells were harvested and counted on the cell counter. Cells were then divided for flow analysis and the remaining cells were lysed with RLTbuffer and stored at -80°C for RNA extraction and gene expression. Supernatants were aliquoted and frozen at -20°C for immunoglobulin assays.

#### 8.4.4 Preparation of Test Compound Stock Solutions and Dilutions

The test compounds was weighed and dissolved in sterile 100% DMSO (dimethyl sulfoxide; Research Organics, Cleveland, OH) to create 40 mM stock solution. Dilutions of the 40 mM stock were used in the assay to obtain final test compound concentrations based on experimental design.

#### 8.4.5 RNA Extraction and Gene Expression

Differentiated B cells (see section 4.3.3) were harvested for total ribonucleic acid (RNA) preparation with a Qiacube RNA extraction instrument (Qiagen, Valencia, CA) using QIAGEN RNeasy mini spin-column kits. Purified RNA was reverse transcribed into cDNA with thermal cycler [MJ Research; Inc., St. Bruno, Quebec, Canada) using a reverse-transcriptase kit (Applied Biosystems). The gene expression assay was carried out using 7500 RT-PCR system (Applied Biosystems) in triplicate. A glyceraldehyde 3-phosphate dehydrogenase gene expression assay control was run for each sample and used as a normalization control. For each gene, samples within each experiment were normalized to 0.1% DMSO treatment only for that particular time point.

Supernatants (from section 8.4.3) were harvested and analyzed by ELISA for IgG and IgM production (ZeptoMetrix Corp. Buffalo, NY).

#### 8.4.6 Cell Phenotyping

Differentiated B cells (see section 4.3.3) were harvested, counted, and aliquoted at about $1 \times 10^6$ cells or less per 4 mL tube. The cells were washed 1X with stain buffer. Next, the cells then were blocked with 10% human serum/PBS

for 20-30 minutes. Following blocking, the cells were centrifuged for 5 minutes at 1200 rpm and supernatants discarded. In the 100 μL of remaining buffer, 20 μL of various BD Pharmigen flow antibodies were added according to experimental design. The cells were stained for 20-30 minutes at 4°C. Then the cells were washed 2X with stain buffer and supernatants discarded. Next, 500 μL of stain buffer or PBS was added to the tubes. The samples were immediately analyzed or put at 4°C overnight. Cells were stained with mouse anti-human CD20 and CD38, CD19 and CD27, or respective isotype controls. All samples were analyzed using a FACSCanto flow cytometer, FACSDiva analysis software (BD Bioscience), and FlowJo Analysis software.

### 8.4.7 Cell Viability Analysis

To determine live cell count, B cells (see section 4.3.3) were stained with 0.4% trypan blue and live cells counted using the Countess automated cell counter (Invitrogen) in duplicate samples.

The data was graphed using GraphPad Prism 5.0 software. $IC_{50}$ values were calculated using non-linear regression, sigmoidal-dose response constraining the top to 100% and bottom to 0% allowing for a variable slope. The results for test compounds in the Ig assays were expressed as the percentage inhibition relative to control DMSO values.

The potency for inhibition of normal and SLE PBMC production of IgG and IgM for the test compound is as follows:

| IgG $IC_{50}$ (nM) | | IgM $IC_{50}$ (nM) | |
|---|---|---|---|
| SLE (n=3) | Normal (n=3) | SLE (n=3) | Normal (n=3) |
| 46 | 61 | 30 | 23 |

### 8.5 EXAMPLE 5: EFFECTS ON PREVENTION AND TREATMENT OF BLEOMYCIN INDUCED DERMAL FIBROSIS

In this example, the effects of 3-(5-amino-2-methyl-4-oxoquinazolin-3(4*H*)-yl)piperidine-2,6-dione (test compound) on the progression of experimental fibrosis and the regression of established fibrosis in a mouse model of bleomycin-induced dermal fibrosis was studied.

The following abbreviations are used in this example:

| Abbreviation or Specialist Term | Explanation or Definition |
|---|---|
| ANOVA | Analysis of Variance |
| α-SMA | Alpha Smooth Muscle Actin |
| CMC | Carboxymethyl Cellulose |
| ECM | Extracellular Matrix |
| NaCl | Sodium Chloride |
| PO | orally |
| QD | Once daily dosing |
| SSc | Systemic Sclerosis |

DBA/2 mice were used in this study. Eight animals were used per treatment group in the study.

Mice were kept in the animal house under standard conditions with food and water ad libidum.

The vehicle, 0.5% carboxymethyl cellulose (CMC)/0.25% Tween 80, was prepared in distilled $H_20$ and dissolved overnight on a magnetic stirrer (add 0.5g CMC; Sigma #C9481) and 0.25ml Tween 80 (Sigma #P8074) to 99.75 ml to make a total of 100 ml 0.5% CMC/0.25% Tween 80).

The test compound powder was weighed out and suspended fresh daily in the vehicle 0.5% CMC/0.25% Tween 80, to avoid drug hydrolysis in the aqueous medium. The compound was suspended, not dissolved, in this vehicle. The formulation was homogenized with a Teflon pestle and mortar (Potter-Elvehjem tissue grinder) using a motorized Eberbach tissue homogenizer. The daily drug stock concentration used in these studies was 3 mg/ml.

Bleomycin was obtained from the pharmacy of the University of Erlangen-Nuremberg and freshly prepared once a week. Skin fibrosis was induced in 6-week-old DBA mice by local intracutaneous injections of 100 μl of bleomycin dissolved in 0.9% NaCl, at a concentration of 0.5 mg/ml, every other day in defined areas of 1.5 cm2 on the upper back.

**Study Design**

The mouse model of bleomycin induced dermal fibrosis is widely used to evaluate anti-fibrotic therapeutics. In this model, a localized dermal fibrosis is induced by intradermal injections with bleomycin every other day for 3 weeks. This model resembles early, inflammatory stages of SSc. To evaluate potential effects on prevention of fibrosis, treatment was initiated simultaneously with the first bleomycin injection. To study the effect of test compound on prevention of bleomycin-induced dermal fibrosis *in vivo,* the treatments were divided into following groups:

- Control group: Intradermal injection of NaCl for 3 weeks. Treatment consisted of administration of the vehicle (0.5% CMC/0.25% Tween 80).
- Untreated bleomycin group: Intradermal injection of bleomycin for three weeks. Administration of the vehicle (0.5% CMC/0.25% Tween 80).
- Test compound group: Intradermal injection of bleomycin for three weeks. The test compound was administered at 30 mg/kg; PO, QD.
- Positive control group: Intradermal injection of bleomycin for three weeks. Injection of Imatinib (50 mg/kg; IP, QD). Imatinib mesylate has previously been shown to exert potent anti-fibrotic effects in bleomycin induced dermal fibrosis. *See* Akhmetshina A. et al., Arthritis Rheum 2009; 60(1):219-224.

To evaluate regression of fibrosis, a modified model of bleomycin induced dermal fibrosis was used. Mice were pre-challenged with bleomycin to induce a robust skin fibrosis. One group received treatment with the test compound, while challenge with bleomycin was ongoing for additional three weeks. The outcome of this group was compared to mice challenged with bleomycin for six weeks (prevention of further progression) and to mice challenged with bleomycin for three weeks followed by NaCl for additional three weeks (induction of regression). The following groups were used in the regression study:

- Control group: Intradermal injection of NaCl for six weeks. Control treatment consisted of administration of the vehicle.

- Untreated bleomycin group 1 (regression): Intradermal injection of bleomycin for three weeks followed by intradermal injections of NaCl for another three weeks. Treatment consisted of administration of the vehicle. Untreated bleomycin group 2 (prevention of progression): Intradermal injection of bleomycin for six weeks. Treatment consisted of administration of the vehicle.

- Test compound group: Intradermal injection of bleomycin for six weeks. The test compound was administered at 30 mg/kg; PO, QD.

- Positive control group: Intradermal injection of bleomycin for six weeks. Injection of Imatinib (50 mg/kg; IP, QD)

**Experimental Procedure**

Dermal thickness was determined by staining with hematoxylin and eosin and activated fibroblasts by using immunohistochemistry for alpha smooth mucle actin ($\alpha$-SMA). The dermal thickness, as determined by the modified Rodnan Skin Score, is currently the most common primary outcome in human clinical trials for anti-fibrotic agents in SSc. Skin sections were stained with hematoxylin/eosin for better visualization of the tissue structure. Dermal thickness was analyzed with a Nikon Eclipse 80i microscope (Nikon, Badhoevedorp, The Netherlands) by measuring the maximal distance between the epidermal-dermal junction and the dermal-subcutaneous fat junction at 4 different skin sections in each mouse. The evaluation was performed by 2 independent examiners.

For quantification of myofibroblasts, skin sections were deparaffinized and incubated with 5% bovine serum albumin for 60 minutes. Cells positive for $\alpha$-SMA were detected by incubation with monoclonal anti-$\alpha$-SMA antibodies (clone 1A4; Sigma-Aldrich, Steinheim, Germany) for 2 hours at room temperature followed by incubation with 3% hydrogen peroxide for 10 minutes. Goat anti-rabbit antibodies labeled with horseradish peroxidase (Dako, Hamburg, Germany) were used as secondary antibodies. The expression of $\alpha$-SMA was visualized with 3,3'-diaminobenzidine tetrahydrochloride (Sigma-Aldrich). Monoclonal mouse IgG antibodies (Calbiochem, San Diego, CA) were used as controls.

In addition, the amount of collagen in lesional skin will be measured with the SirCol collagen assay; RNA and plasma of all mice were saved for further analyses.

The test compound significantly decreases dermal thickness of lesional skin in the bleomycin dermal fibrosis mouse model. The test compound at 30 mg/kg; PO, QD significantly prevented dermal thickening by approximately 22 $\pm$ 0.49 % ($p < 0.0001$).

Representative photomicrographs of hematoxylin and eosin stained skin sections are shown in FIG. 8. Dermal thickness was assessed by measuring the maximal distance between the epidermal-dermal junction and the dermal-subcutaneous fat junction. The line drawn between the junction points shows the relative thickness in the treatment groups.

To determine the effect of the treatments on fibroblast activation, α-SMA + myofibroblasts were counted in lesional skin sections. The test compound at 30 mg/kg; PO, QD reduced the number of myofibroblasts by 30 ± 0.23 % ($p < 0.0001$).

**Effect on the regression of bleomycin induced dermal fibrosis**

The inhibitory effects of the test compound on progression of fibrosis were also confirmed in the modified bleomycin model designed to investigate potential regression of fibrosis. The test compound reduced dermal thickness of bleomycin induced dermal thickening by 22 ± 0.28 % ($p < 0.0001$). FIG. 9 shows photomicrographs of representative hematoxylin and eosin stained skin sections. Dermal thickness was assessed by measuring the maximal distance between the epidermal-dermal junction and the dermal-subcutaneous fat junction. The line drawn between the junction points shows relative thickness in the treatment groups.

The present invention is not to be limited in scope by the specific embodiments described herein. Indeed, various modifications of the invention in addition to those described will become apparent to those skilled in the art from the foregoing description and accompanying figures. Such modifications are intended to fall within the scope of the appended claims.

## Claims

1. A compound for use in a method for treating, preventing or managing an immune-related disease or an inflammatory disease, wherein the method comprises administering to a patient in need thereof an effective amount of the compound, wherein the compound is 3-(5-amino-2-methyl-4-oxoquinazolin-3(4H)-yl)piperidine-2,6-dione, or a pharmaceutically acceptable salt, solvate, hydrate, stereoisomer, tautomer, racemic mixture, co-crystal, clathrate, or polymorph thereof.

2. The compound for use of claim 1, wherein the disease is systemic lupus erythematosus, scleroderma, Sjögren syndrome, ANCA-induced vasculitis, anti-phospholipid syndrome or myasthenia gravis.

3. The compound for use of claim 1 or 2, wherein the disease is systemic lupus erythematosus.

4. The compound for use of any of claims 1-3, wherein the disease is severe systemic lupus erythematosus.

**5.** The compound for use of claim 1 or 2, wherein the disease is scleroderma.

**6.** The compound for use of claim 5, wherein the scleroderma is localized, systemic, limited or diffuse scleroderma.

**7.** The compound for use of claim 6, wherein the systemic scleroderma comprises CREST syndrome.

**8.** The compound for use of claim 1, wherein the disease is systemic lupus erythematosus, and the method comprises administering an effective amount of said compound to a patient having the symptom of systemic lupus erythematosus, wherein the symptom is selected from the group consisting of joint pain, joint swelling, arthritis, chest pain when taking a deep breath, fatigue, fever with no other cause, general discomfort, uneasiness, hair loss, mouth sores, swollen lymph nodes, sensitivity to sunlight, skin rash, headaches, numbness, tingling, seizures, vision problems, personality changes, abdominal pain, nausea, vomiting, abnormal heart rhythms, coughing up blood and difficulty breathing, patchy skin color and Raynaud's phenomenon.

**9.** The compound for use of claim 1, wherein the disease is scleroderma, and the method comprises administering to a patient having the symptom of scleroderma an effective amount of said compound, wherein the symptom is selected from the group consisting of

(i) gradual hardening, thickening, and tightening of the skin;
(ii) skin discoloration;
(iii) numbness of extremities; (iv) shiny skin; (v) small white lumps under the surface of the skin that erupt into a chalky white fluid; (vi) Raynaud's esophagaeal dysfunction; (vii) telangiectasia; (viii) pain and/or stiffness of the joints; (ix) swelling of the hands and feet; (x) itching of the skin; (xi) stiffening and curling of the fingers;
(xii) ulcers on the outside of certain joints, such as knuckles and elbows; (xiii) digestive problems, such as heartburn, difficulty in swallowing, diarrhea, irritable bowel, and constipation; (xiv) fatigue and weakness; (xv) shortness of breath; (xvi) arthritis; (xvii) hair loss; (xviii) internal organ problems; (xix) digital ulcers; and (xx) digital auto-amputation.

**10.** The compound for use of claim 1, wherein the method comprises improving the modified Rodnan skin score, reducing or improving the skin thickness, reducing or improving skin induration, improving the pulmonary function, improving the dermatology quality of life index, improving the carbon monoxide diffusing capacity, improving the Mahler Dyspnea index, improving the Saint George's Respiratory Questionnaire score, improving the UCLA scleroderma clinical trial consortium gastrointestinal tract score, improving flow-mediated dilatation or improving or increasing the six minute walk distance of a patient having scleroderma.

**11.** The compound for use of any of claims 1-10 further comprising administering a second active agent being an anti-inflammatory or immunomodulatory compound.

**12.** The compound for use of any of claims 1-11, wherein the effective amount of said compound is about 0.005 mg/kg to about 10 mg/kg of the patient's body weight.

**13.** An *in vitro* method for modulating activity of a B cell comprising contacting the cell with an effective amount of 3-(5-amino-2-methyl-4-oxoquinazolin-3(4H)-yl)piperidine-2,6-dione or a pharmaceutically acceptable salt, solid form, solvate, hydrate, stereoisomer, tautomer, racemic mixture, co-crystal, clathrate, or polymorph thereof.

**14.** An *in vitro* method for modulating activity of a T cell comprising contacting the cell with an effective amount of 3-(5-amino-2-methyl-4-oxoquinazolin-3(4H)-yl)piperidine-2,6-dione or a pharmaceutically acceptable salt, solid form, solvate, hydrate, tautomer, stereoisomer, racemic mixture, co-crystal, clathrate, or polymorph thereof.

## Patentansprüche

**1.** Verbindung für die Verwendung in einem Verfahren zur Behandlung, Prävention oder Managen einer immun-bezogenen Erkrankung oder einer Entzündungserkrankung, wobei das Verfahren das Verabreichen einer wirksamen Menge der Verbindung an einen betroffenen Patienten umfasst, wobei die Verbindung 3-(5-Amino-2-methyl-4-oxochinazolin-3(4H)-yl)piperidin-2,6-dion oder ein/eine pharmazeutisch annehmbares/annehmbare Salz, Solvat, Hydrat, Stereoisomer, Tautomer, racemische Mischung, Co-Kristall, Clathrat oder Polymorph davon ist.

2. Verbindung für die Verwendung nach Anspruch 1, wobei die Erkrankung systemischer Lupus erythematodes, Sklerodermie, Sjögren Syndrom, ANCAinduzierte Vaskulitis, anti-Phospholipid-Syndrom oder Myasthenia gravis ist.

3. Verbindung für die Verwendung nach Anspruch 1 oder 2, wobei die Erkrankung systemischer Lupus erythematodes ist.

4. Verbindung für die Verwendung nach einem der Ansprüche 1-3, wobei die Erkrankung schwerer systemischer Lupus erythematodes ist.

5. Verbindung für die Verwendung nach Anspruch 1 oder 2, wobei die Erkrankung Sklerodermie ist.

6. Verbindung für die Verwendung nach Anspruch 5, wobei die Sklerodermie lokalisierte, systemische, begrenzte oder diffuse Sklerodermie ist.

7. Verbindung für die Verwendung nach Anspruch 6, wobei die systemische Sklerodermie das CREST Syndrom umfasst.

8. Verbindung für die Verwendung nach Anspruch 1, wobei die Erkrankung systemischer Lupus erythematodes ist und das Verfahren das Verabreichen einer wirksamen Menge der Verbindung an einen Patienten mit dem Symptom von systemischem Lupus erythematodes umfasst, wobei das Symptom ausgewählt ist aus der Gruppe bestehend aus Gelenkschmerz, Gelenkschwellung, Arthritis, Brustschmerzen beim tiefen Einatmen, Müdigkeit, Fieber ohne anderen Grund, allgemeinem Unwohlsein, Unruhe, Haarausfall, Mundentzündungen, geschwollenen Lumphknoten, Empfindlichkeit gegenüber Sonnenlicht, Hautausschlag, Kopfschmerzen, Taubheitsgefühl, Kribbeln, Anfällen, Sehproblemen, Persönlichkeitsveränderungen, abdominalem Schmerz, Übelkeit, Erbrechen, abnormalem Herzrhythmus, Bluthusten und Schwierigkeiten beim Atmen, ungleichmäßiger Hautfarbe und Raynaud Syndrom.

9. Verbindung für die Verwendung nach Anspruch 1, wobei die Erkrankung Sklerodermie ist und das Verfahren das Verabreichen einer wirksamen Menge der Verbindung an einen Patienten mit dem Symptom von Sklerodermie umfasst, wobei das Symptom ausgewählt ist aus der Gruppe bestehend aus (i) gradueller Verhärtung, Verdickung und Straffung der Haut; (ii) Hautverfärbung; (iii) Taubheit der Extremitäten; (iv) glänzender Haut; (v) kleine weiße Klümpchen unter der Oberfläche der Haut, die in kreideweiße Flüssigkeit ausbrechen; (vi) Raynaud ösophageale Dysfunktion; (vii) Telangiektasie; (viii) Schmerz und/oder Steifheit der Gelenke; (ix) Schwellung der Hände und Füße; (x) Jucken der Haut; (xi) Steifheit und Krümmung der Finger; (xii) Geschwüre an der Außenseite bestimmter Gelenke, wie zum Beispiel Knöchel und Ellbogen; (xiii) Verdauungsproblemen, wie zum Beispiel Sodbrennen, Schwierigkeiten beim Schlucken, Durchfall, Reizdarm und Verstopfung; (xiv) Müdigkeit und Schwäche; (xv) Kurzatmigkeit; (xvi) Arthritis; (xvii) Haarausfall; (xviii) internen Organproblemen; (xix) digitalen Geschwüren; und (xx) digitaler Autoamputation.

10. Verbindung für die Verwendung nach Anspruch 1, wobei das Verfahren die Verbesserung des modifizierten Rodnan Skin Score, Verringerung oder Verbesserung der Hautdicke, Verringerung oder Verbesserung der Hautinduration, Verbesserung der Lungenfunktion, Verbesserung des dermatologischen Index der Lebensqualität, Verbesserung der Kohlenmonoxid-Diffusionskapazität, Verbesserung des Mahler Dyspnea Index, Verbesserung des Saint George's Respiratory Questionnaire Score, Verbesserung des UCLA Sklerodermie Clinical Trial Consortium Gastrointestinal Tract Score, Verbesserung der flussvermittelten Dilatation oder Verbesserung oder Erhöhung der 6-Minuten-Gehdistanz eines Patienten mit Sklerodermie umfasst.

11. Verbindung für die Verwendung nach einem der Ansprüche 1-10 weiter umfassend das Verabreichen eines zweiten Wirkstoffs, der eine entzündungshemmende oder immunomodulatorische Verbindung ist.

12. Verbindung für die Verwendung nach einem der Ansprüche 1-11, wobei die wirksame Menge der Verbindung ungefähr 0.005 mg/kg bis ungefähr 10 mg/kg des Körpergewichts des Patienten ist.

13. *In vitro* Verfahren zur Modulation der Aktivität einer B-Zelle umfassend das In-Kontakt-bringen der Zelle mit einer wirksamen Menge von 3-(5-Amino-2-methyl-4-oxochinazolin-3(4H)-yl)piperidin-2,6-dion oder einem/einer pharmazeutisch annehmbaren Salz, festen Form, Solvat, Hydrat, Stereoisomer, Tautomer, racemischen Mischung, Co-Kristall, Clathrat oder Polymorph davon.

14. *In vitro* Verfahren zur Modulation der Aktivität einer T-Zelle umfassend das In-Kontakt-bringen der Zelle mit einer

wirksamen Menge von 3-(5-Amino-2-methyl-4-oxochinazolin-3(4H)-yl)piperidin-2,6-dion oder einem/einer pharmazeutisch annehmbaren Salz, festen Form, Solvat, Hydrat, Stereoisomer, Tautomer, racemischen Mischung, Co-Kristall, Clathrat oder Polymorph davon.

## Revendications

1. Composé à utiliser dans un procédé pour le traitement, la prévention ou la gestion d'une maladie liée au système immunitaire ou d'une maladie inflammatoire, dans lequel le procédé comprend l'administration à un patient en ayant besoin d'une quantité efficace du composé, dans lequel le composé est une 3-(5-amino-2-méthyl-4-oxoquinazolin-3(4H)-yl)pipéridine-2,6-dione, ou un sel acceptable d'un point de vue pharmaceutique, solvate, hydrate, stéréoisomère, tautomère, mélange racémique, cocristal, clathrate, ou polymorphe de cette dernière.

2. Composé à utiliser selon la revendication 1, dans lequel la maladie est le lupus érythémateux systémique, la sclérodermie, le syndrome de Sjögren, la vascularite induite par des ANCA, le syndrome des antiphospholipides ou la myasthénie grave.

3. Composé à utiliser selon la revendication 1 ou 2, dans lequel la maladie est le lupus érythémateux systémique.

4. Composé à utiliser selon l'une quelconque des revendications 1-3, dans lequel la maladie est le lupus érythémateux systémique sévère.

5. Composé à utiliser selon la revendication 1 ou 2, dans lequel la maladie est la sclérodermie.

6. Composé à utiliser selon la revendication 5, dans lequel la sclérodermie est une sclérodermie localisée, systémique, limitée ou diffuse.

7. Composé à utiliser selon la revendication 6, dans lequel la sclérodermie systémique comprend le syndrome de CREST.

8. Composé à utiliser selon la revendication 1, dans lequel la maladie est le lupus érythémateux systémique, et le procédé comprend l'administration d'une quantité efficace dudit composé à un patient présentant le symptôme du lupus érythémateux systémique, dans lequel le symptôme est sélectionné dans le groupe constitué d'une douleur articulaire, un gonflement articulaire, l'arthrite, une douleur thoracique lors d'une inspiration profonde, une fatigue, une fièvre sans autre cause, un malaise général, un mal-être, une chute des cheveux, des plaies à la bouche, des noeuds lymphatiques enflés, une sensibilité à la lumière du soleil, une éruption cutanée, des céphalées, un engourdissement, des picotements, des crises épileptiques, des problèmes de vision, des changements de personnalité, une douleur abdominale, des nausées, des vomissements, des arythmies cardiaques, des expectorations de sang et des difficultés à respirer, une couleur de peau inégale et un phénomène de Raynaud.

9. Composé à utiliser selon la revendication 1, dans lequel la maladie est la sclérodermie et le procédé comprend l'administration à un patient présentant le symptôme de sclérodermie d'une quantité efficace dudit composé, dans lequel le symptôme est sélectionné dans le groupe constitué de : (i) le durcissement, l'épaississement et le resserrement progressifs de la peau ; (ii) une décoloration de la peau ; (iii) un engourdissement des extrémités ; (iv) une peau brillante ; (v) des petites masses blanches sous la surface de la peau qui éclatent en un fluide blanc crayeux ; (vi) un dysfonctionnement oesophagien de Raynaud ; (vii) une télangiectasie ; (viii) une douleur et/ou une rigidité des articulations ; (ix) un gonflement des mains et des pieds ; (x) des démangeaisons de la peau ; (xi) une raideur et une torsion des doigts ; (xii) des ulcères à l'extérieur de certaines articulations, comme les jointures et les coudes ; (xiii) des problèmes digestifs, comme des brûlures d'estomac, une difficulté à avaler, des diarrhées, l'intestin irritable et la constipation ; (xiv) une fatigue et une faiblesse ; (xv) un essoufflement ; (xvi) une arthrite ; (xvii) une chute des cheveux ; (xviii) des problèmes d'organes internes ; (xix) des ulcères digitaux ; et (xx) une auto-amputation digitale.

10. Composé à utiliser selon la revendication 1, dans lequel le procédé comprend l'amélioration du score de Rodnan modifié pour la peau, la réduction ou l'amélioration de l'épaisseur de la peau, la réduction ou l'amélioration de l'induration de la peau, l'amélioration de la fonction pulmonaire, l'amélioration de l'index de qualité de vie en dermatologie, l'amélioration de la capacité de diffusion du monoxyde de carbone, l'amélioration de l'index de dyspnée de Mahler, l'amélioration du score du Questionnaire Respiratoire de Saint Georges, l'amélioration du score pour le tube digestif mis au moins par le UCLA Scleroderma Clinical Trial Consortium, l'amélioration de la dilatation médiée

par le flux sanguin ou l'amélioration ou l'augmentation de la distance de marche à six minutes d'un patient souffrant de sclérodermie.

**11.** Composé à utiliser selon l'une quelconque des revendications 1-10 comprenant en outre l'administration d'un second agent actif qui est un composé anti-inflammatoire ou immunomodulateur.

**12.** Composé à utiliser selon l'une quelconque des revendications 1-11, dans lequel la quantité efficace dudit composé est d'environ 0,005 mg/kg à environ 10 mg/kg de poids corporel du patient.

**13.** Procédé *in vitro* pour moduler une activité d'une cellule B comprenant la mise en contact de la cellule avec une quantité efficace de 3-(5-amino-2-méthyl-4-oxoquinazolin-3(4H)-yl)pipéridine-2,6-dione ou d'un sel acceptable d'un point de vue pharmaceutique, forme solide, solvate, hydrate, stéréoisomère, tautomère, mélange racémique, cocristal, clathrate, ou polymorphe de cette dernière.

**14.** Procédé *in vitro* pour moduler une activité d'une cellule T comprenant la mise en contact de la cellule avec une quantité efficace de 3-(5-amino-2-méthyl-4-oxoquinazolin-3(4H)-yl)pipéridine-2,6-dione ou d'un sel acceptable d'un point de vue pharmaceutique, forme solide, solvate, hydrate, tautomère, stéréo-isomère, mélange racémique, cocristal, clathrate, ou polymorphe de cette dernière.

FIG. 1

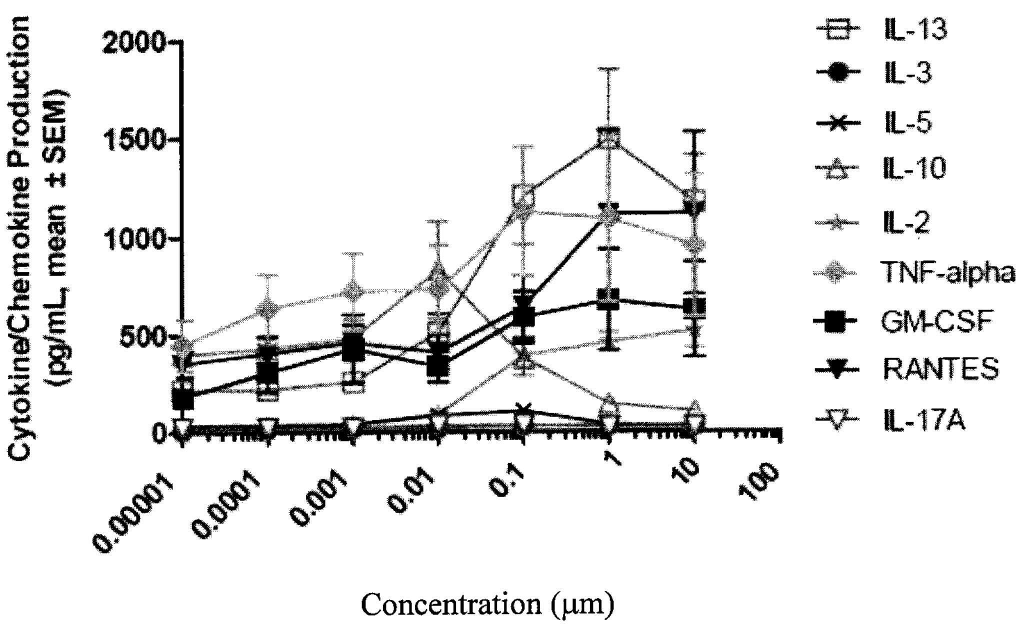
Cytokine/Chemokine Production (pg/mL, mean ± SEM)
2000
1500
1000
500
0
0.00001
0.0001
0.001
0.01
0.1
1
10
100
IL-13
IL-3
IL-5
IL-10
IL-2
TNF-alpha
GM-CSF
RANTES
IL-17A
Concentration (μm)

FIG. 2

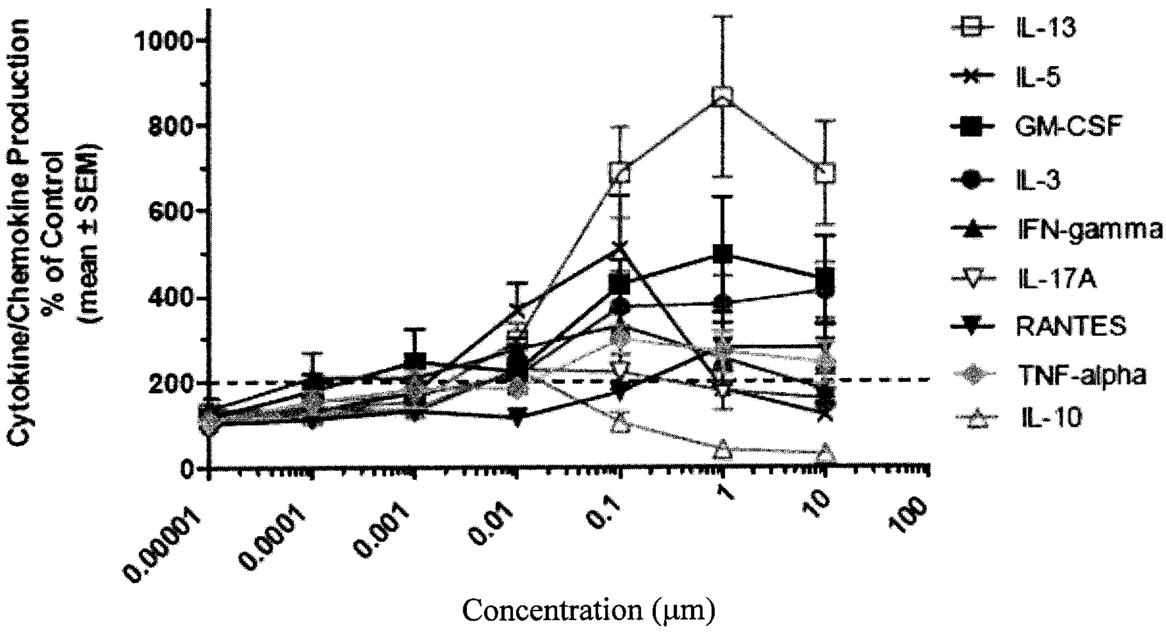
Cytokine/Chemokine Production
% of Control
(mean ± SEM)
1000
800
600
400
200
0
0.00001
0.0001
0.001
0.01
0.1
1
10
100
Concentration (μm)
IL-13
IL-5
GM-CSF
IL-3
IFN-gamma
IL-17A
RANTES
TNF-alpha
IL-10

FIG. 3

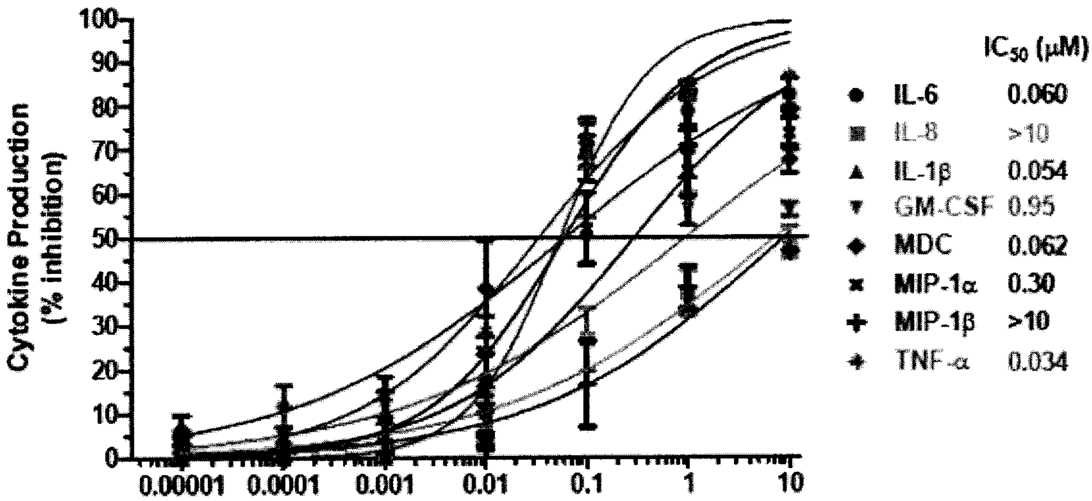


Concentration (µm)

FIG. 4

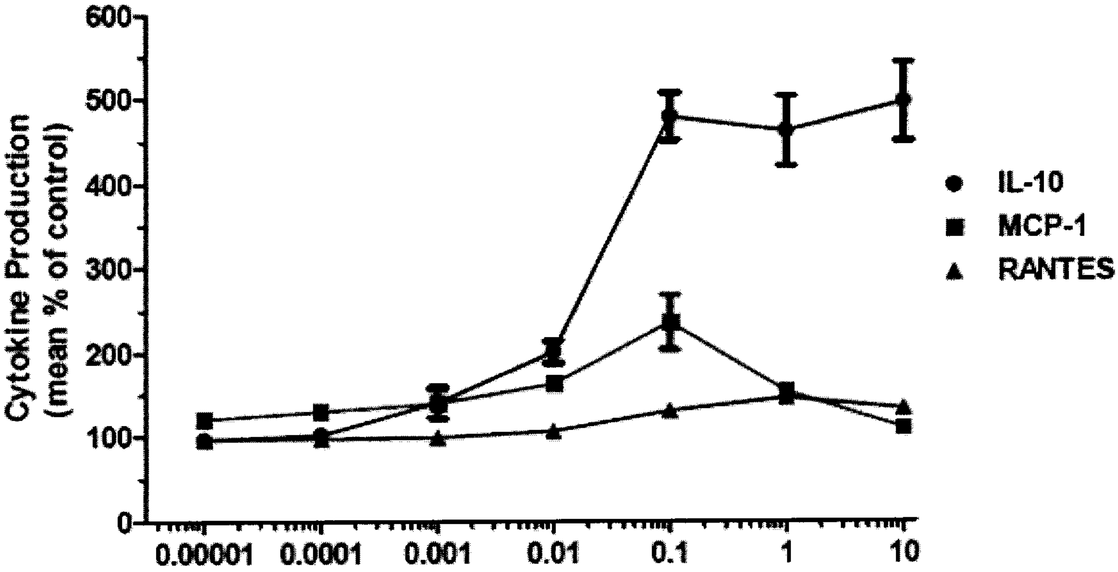


Concentration (μm)

FIG. 5

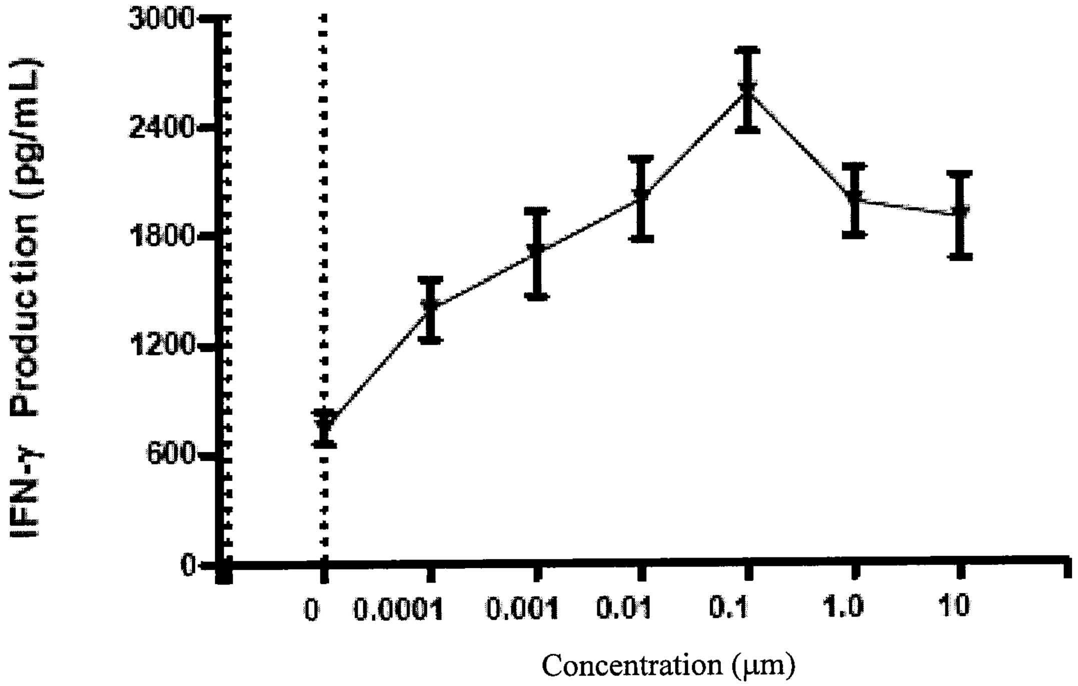
IFN-γ Production (pg/mL)
3000
2400
1800
1200
600
0
0
0.0001
0.001
0.01
0.1
1.0
10
Concentration (μm)

FIG. 6

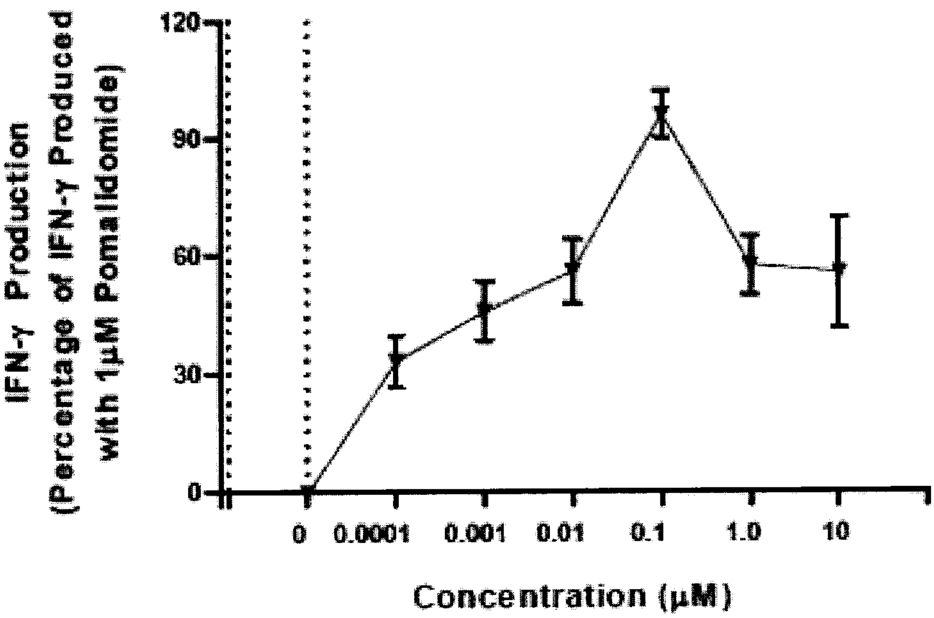
IFN-γ Production
(Percentage of IFN-γ Produced with 1μM Pomalidomide)
120
90
60
30
0
0
0.0001
0.001
0.01
0.1
1.0
10
Concentration (μM)

FIG. 7

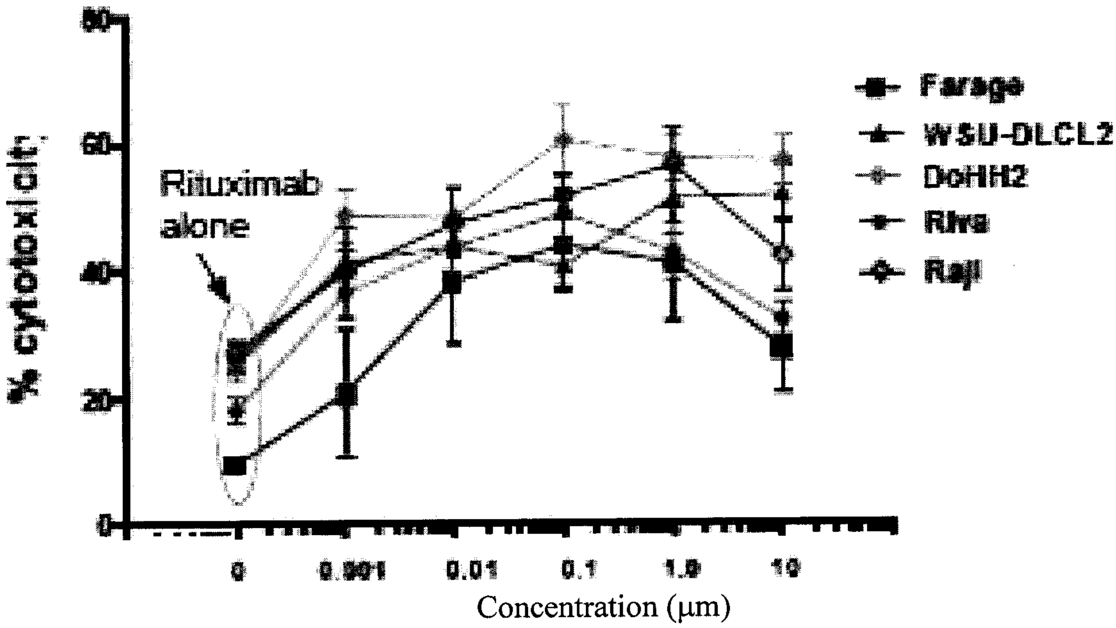
% cytotoxicity
Rituximab alone
0
20
40
60
80
0
0.001
0.01
0.1
1.0
10
Concentration (μm)
Farage
WSU-DLCL2
DoHH2
Riva
Raji

FIG. 8

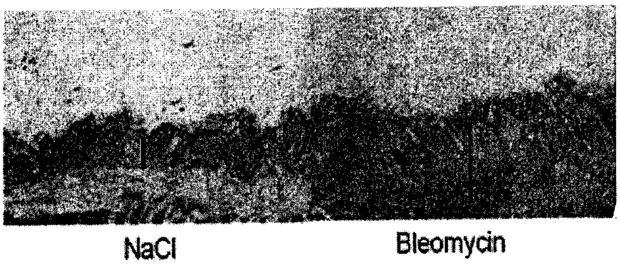

NaCl Bleomycin

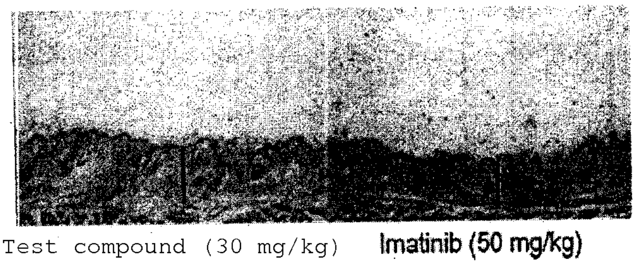

Test compound (30 mg/kg) Imatinib (50 mg/kg)

FIG. 9

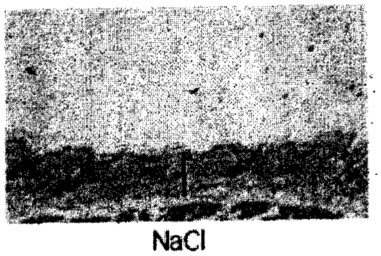
NaCl

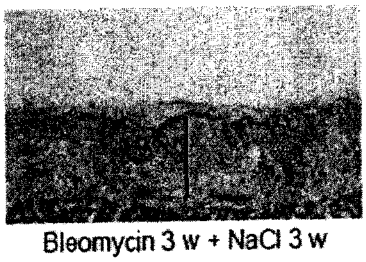
Bleomycin 3 w + NaCl 3 w

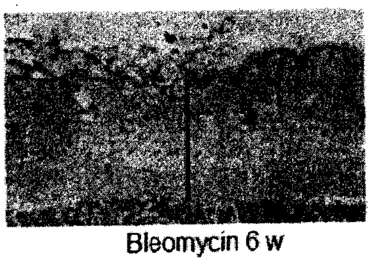
Bleomycin 6 w

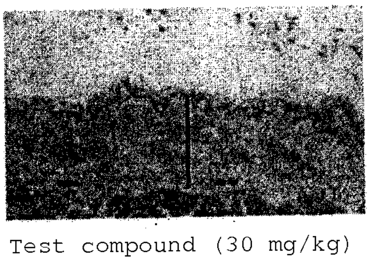
Test compound (30 mg/kg)

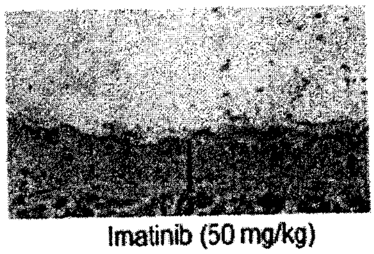
Imatinib (50 mg/kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61451995 A
- US 61480272 A
- US 7635700 B
- US 61451806 B
- US 61451806 A
- WO 2002088171 A
- WO 2006055689 A
- WO 2002032925 A
- WO 2005037989 A
- US 20030133939 A
- US 20050238646 A
- US 3845770 A
- US 3916899 A
- US 3536809 A
- US 3598123 A
- US 4008719 A
- US 5674533 A
- US 5059595 A
- US 5591767 A
- US 5120548 A
- US 5073543 A
- US 5639476 A
- US 5354556 A
- US 5639480 A
- US 5733566 A
- US 5739108 A
- US 5891474 A
- US 5922356 A
- US 5972891 A
- US 5980945 A
- US 5993855 A
- US 6045830 A
- US 6087324 A
- US 6113943 A
- US 6197350 B
- US 6248363 B
- US 6264970 B
- US 6267981 B
- US 6376461 B
- US 6419961 B
- US 6589548 B
- US 6613358 B
- US 6699500 B
- US 5134127 A
- WO 61451995 A
- WO 61480272 A


### Non-patent literature cited in the description


- **WALLACE.** The Lupus Book: A Guide for Patients and Their Families. Oxford University Press, 2000
- **HACHULLA E ; LAUNAY D.** Diagnosis and classification of systemic sclerosis. *Clin Rev Allergy Immunol,* 2010, vol. 40 (2), 78-83
- **JACQUES, J. et al.** Enantiomers, Racemates and Resolutions. Wiley-Interscience, 1981
- **WILEN, S. H. et al.** *Tetrahedron,* 1977, vol. 33, 2725
- **ELIEL, E. L.** Stereochemistry of Carbon Compounds. McGraw-Hill, 1962
- **WILEN, S. H.** Tables of Resolving Agents and Optical Resolutions. Univ. of Notre Dame Press, 1972, 268
- Systemic Lupus Erythematosus. **HAHN BH.** Harrison's Principles of Internal Medicine. McGraw-Hill, 2005, 1960-1967
- Remington's Pharmaceutical Sciences. Mack Publishing, 2000
- **JENS T. CARSTENSEN.** Drug Stability: Principles & Practice. Marcel Dekker, 1995, 379-80
- **SEFTON.** *CRC Crit. Ref. Biomed. Eng.,* 1987, vol. 14, 201
- **BUCHWALD et al.** *Surgery,* 1980, vol. 88, 507
- **SAUDEK et al.** *N. Engl. J. Med.,* 1989, vol. 321, 574
- **GOODSON.** *Medical Applications of Controlled Release,* 1984, vol. 2, 115-138
- **LANGER.** *Science,* 1990, vol. 249, 1527-1533
- Remington's Pharmaceutical Sciences. Mack Publishing, 1980
- Introduction to Pharmaceutical Dosage Forms. Lea & Febiger, 1985
- **AKHMETSHINA A. et al.** *Arthritis Rheum,* 2009, vol. 60 (1), 219-224

SEQUENCE LISTING

<110> Celgene Corporation,
Anita Gandhi
Peter H. Schafer

<120> USE OF 3-(5-AMINO-2-METHYL-4-OXOQUINAZOLIN-3(4H)-YL)PIPERIDINE-2,6-DIONE IN TREATMENT OF IMMUNE-RELATED AND
INFLAMMATORY DISEASES

<130> 12827-263-228

<140>
<141>

<<150> 61/451,995
<151> 2011-03-11

<150> 61/480,272
<151> 2011-04-28

<160> 2

<170> FastSEQ for Windows Version 4.0

<210> 1
<211> 344
<212> PRT
<213> Artificial Sequence

<220>
<223> human ActRIIA fused to a human Fc domain

<400> 7

```
Ile Leu Gly Arg Ser Glu Thr Gln Glu Cys Leu Phe Phe Asn Ala Asn
 1               5                  10                  15
Trp Glu Lys Asp Arg Thr Asn Gln Thr Gly Val Glu Pro Cys Tyr Gly
            20                  25                  30
Asp Lys Asp Lys Arg Arg His Cys Phe Ala Thr Trp Lys Asn Ile Ser
        35                  40                  45
Gly Ser Ile Glu Ile Val Lys Gln Gly Cys Trp Leu Asp Asp Ile Asn
    50                  55                  60
Cys Tyr Asp Arg Thr Asp Cys Val Glu Lys Lys Asp Ser Pro Glu Val
65                  70                  75                  80
Tyr Phe Cys Cys Cys Glu Gly Asn Met Cys Asn Glu Lys Phe Ser Tyr
                85                  90                  95
Phe Pro Glu Met Glu Val Thr Gln Pro Thr Ser Asn Pro Val Thr Pro
            100                 105                 110
Lys Pro Pro Thr Gly Gly Gly Thr His Thr Cys Pro Pro Cys Pro Ala
        115                 120                 125
Pro Glu Leu Leu Gly Gly Pro Ser Val Phe Leu Phe Pro Pro Lys Pro
    130                 135                 140
Lys Asp Thr Leu Met Ile Ser Arg Thr Pro Glu Val Thr Cys Val Val
145                 150                 155                 160
Val Asp Val Ser His Glu Asp Pro Glu Val Lys Phe Asn Trp Tyr Val
                165                 170                 175
Asp Gly Val Glu Val His Asn Ala Lys Thr Lys Pro Arg Glu Glu Gln


            180                 185                 190
Tyr Asn Ser Thr Tyr Arg Val Val Ser Val Leu Thr Val Leu His Gln
        195                 200                 205
Asp Trp Leu Asn Gly Lys Glu Tyr Lys Cys Lys Val Ser Asn Lys Ala
    210                 215                 220
Leu Pro Val Pro Ile Glu Lys Thr Ile Ser Lys Ala Lys Gly Gln Pro
225                 230                 235                 240
Arg Glu Pro Gln Val Tyr Thr Leu Pro Pro Ser Arg Glu Glu Met Thr
                245                 250                 255
Lys Asn Gln Val Ser Leu Thr Cys Leu Val Lys Gly Phe Tyr Pro Ser
            260                 265                 270
Asp Ile Ala Val Glu Trp Glu Ser Asn Gly Gln Pro Glu Asn Asn Tyr
        275                 280                 285
Lys Thr Thr Pro Pro Val Leu Asp Ser Asp Gly Ser Phe Phe Leu Tyr
    290                 295                 300
Ser Lys Leu Thr Val Asp Lys Ser Arg Trp Gln Gln Gly Asn Val Phe
305                 310                 315                 320
Ser Cys Ser Val Met His Glu Ala Leu His Asn His Tyr Thr Gln Lys
                325                 330                 335
Ser Leu Ser Leu Ser Pro Gly Lys
            340
```

<210> 2
<211> 335
<212> PRT
<213> Artificial Sequence

<220>
<223> Processed ActRIIB-Fc fusion protein with the N-terminal 6 amino acids of the EC domain deleted and the C-terminal 3 amino acids of the EC domain deleted (amino acids 25-131 of SEQ ID NO:28) and with an L79D mutation

<400> 25

```
Glu Thr Arg Glu Cys Ile Tyr Tyr Asn Ala Asn Trp Glu Leu Glu Arg
 1               5                  10                  15
Thr Asn Gln Ser Gly Leu Glu Arg Cys Glu Gly Glu Gln Asp Lys Arg
            20                  25                  30
Leu His Cys Tyr Ala Ser Trp Arg Asn Ser Ser Gly Thr Ile Glu Leu
        35                  40                  45
Val Lys Lys Gly Cys Trp Asp Asp Asp Phe Asn Cys Tyr Asp Arg Gln
    50                  55                  60
Glu Cys Val Ala Thr Glu Glu Asn Pro Gln Val Tyr Phe Cys Cys Cys
65                  70                  75                  80
Glu Gly Asn Phe Cys Asn Glu Arg Phe Thr His Leu Pro Glu Ala Gly
                85                  90                  95
Gly Pro Glu Val Thr Tyr Glu Pro Pro Pro Thr Gly Gly Gly Thr His
            100                 105                 110
Thr Cys Pro Pro Cys Pro Ala Pro Glu Leu Leu Gly Gly Pro Ser Val
        115                 120                 125
Phe Leu Phe Pro Pro Lys Pro Lys Asp Thr Leu Met Ile Ser Arg Thr
    130                 135                 140
Pro Glu Val Thr Cys Val Val Val Asp Val Ser His Glu Asp Pro Glu
145                 150                 155                 160
Val Lys Phe Asn Trp Tyr Val Asp Gly Val Glu Val His Asn Ala Lys
                165                 170                 175
Thr Lys Pro Arg Glu Glu Gln Tyr Asn Ser Thr Tyr Arg Val Val Ser
            180                 185                 190
Val Leu Thr Val Leu His Gln Asp Trp Leu Asn Gly Lys Glu Tyr Lys
        195                 200                 205
Cys Lys Val Ser Asn Lys Ala Leu Pro Ala Pro Ile Glu Lys Thr Ile
    210                 215                 220


Ser Lys Ala Lys Gly Gln Pro Arg Glu Pro Gln Val Tyr Thr Leu Pro
225                 230                 235                 240
Pro Ser Arg Glu Glu Met Thr Lys Asn Gln Val Ser Leu Thr Cys Leu
                245                 250                 255
Val Lys Gly Phe Tyr Pro Ser Asp Ile Ala Val Glu Trp Glu Ser Asn
            260                 265                 270
Gly Gln Pro Glu Asn Asn Tyr Lys Thr Thr Pro Pro Val Leu Asp Ser
        275                 280                 285
Asp Gly Ser Phe Phe Leu Tyr Ser Lys Leu Thr Val Asp Lys Ser Arg
    290                 295                 300
Trp Gln Gln Gly Asn Val Phe Ser Cys Ser Val Met His Glu Ala Leu
305                 310                 315                 320
His Asn His Tyr Thr Gln Lys Ser Leu Ser Leu Ser Pro Gly Lys
                325                 330                 335
```